# EUROPEAN PATENT SPECIFICATION

(11) **EP 1 067 968 B1**
(45) Date of publication and mention of the grant of the patent: **28.02.2007**
(21) Application number: 98918893.3
(22) Date of filing: 30.04.1998
(51) Int. Cl.: A61K 48/00, C12N 15/09, C12N 5/10, C12N 5/00, C12N 15/63, A01K 67/027, C12N 15/85

(54) **METHODS FOR EVALUATING A COMPOUND FOR ITS EFFECT ON SKIN**
METHODE ZUR BEWERTUNG VON VERBINDUNGEN IN BEZUG AUF IHRES EFFEKTES AUF DIE HAUT
PROCEDE D'EVALUATION D'UN COMPOSE EN FONCTION DE SON ACTION CUTANEE

(30) Priority: 17.12.1997 US 69945 P
(43) Date of publication of application: 17.01.2001
(60) Divisional application: 06026453.8
(73) Proprietor: THE GENERAL HOSPITAL CORPORATION, Boston, MA 02114 (US); SHISEIDO COMPANY LIMITED, Yokohama-shi 103 (JP)
(72) Inventor: BURGESON, Robert, E., Boston, MA 02116 (US); AMANO, Satoshi, Yokohama-shi 103 (JP); KISHIMOTO, Jiro, Yokohama-shi 103 (JP); NISHIYAMA, Toshio, Yokohama-shi 103 (JP); EHAMA, Ritsuko, Yokohama-shi 103 (JP)
(74) Representative: Ruffles, Graham Keith
(86) International application number: PCT/US1998/008794
(87) International publication number: WO 1999/030743

(56) References cited:
- EP-A- 0 633 315
- WO-A-96/37237
- NASO MF, ZIMMERMANN DR, IOZZO RV: "Characterization of the complete genomic structure of the human versican gene and functional analysis of its promoter" JOURNAL OF BIOLOGICAL CHEMISTRY, vol. 269, no. 52, pages 32999-33008, XP002156383
- GILLE JENS ET AL: "Transforming growth factor-alpha-induced transcriptional activation of the vascular permeability factor (VPF/VEGF) gene requires AP-2-dependent DNA binding and transactivation." EMBO (EUROPEAN MOLECULAR BIOLOGY ORGANIZATION) JOURNAL, vol. 16, no. 4, 1997, pages 750-759, XP002164519 ISSN: 0261-4189
- TISCHER E ET AL: "THE HUMAN GENE FOR VASCULAR ENDOTHELIAL GROWTH FACTOR MULTIPLE PROTEIN FORMS ARE ENCODED THROUGH ALTERNATIVE EXON SPLICING" JOURNAL OF BIOLOGICAL CHEMISTRY,US,AMERICAN SOCIETY OF BIOLOGICAL CHEMISTS, BALTIMORE, MD, vol. 266, no. 18, 25 June 1991 (1991-06-25), pages 11947-11954, XP002048804 ISSN: 0021-9258
- ITANO N ET AL: "MOLECULAR CLONING OF HUMAN HYALURONAN SYNTHASE" BIOCHEMICAL AND BIOPHYSICAL RESEARCH COMMUNICATIONS,US,ACADEMIC PRESS INC. ORLANDO, FL, vol. 222, no. 3, 24 May 1996 (1996-05-24), pages 816-821, XP002038786 ISSN: 0006-291X
- RAMIREZ A ET AL: "A 5'-upstream region of a bovine keratin 6 gene confers tissue-specific expression and hyperproliferation-related induction in transgenic mice" PROCEEDINGS OF THE NATIONAL ACADEMY OF SCIENCES OF THE UNITED STATES OF AMERICA, vol. 92, May 1995 (1995-05), pages 4783-4787, XP002156382
- BERNSTEIN E F ET AL: "Evaluation of sunscreens with various sun protection factors in a new transgenic mouse model of cutaneous photoaging that measures elastin promoter activation" JOURNAL OF THE AMERICAN ACADEMY OF DERMATOLOGY,US,C.V. MOSBY, ST. LOUIS, MO, vol. 37, no. 5, PART 01, November 1997 (1997-11), pages 725-729, XP002117296 ISSN: 0190-9622
- MOHAN ROYCE ET AL: "Developmental and wound-inducible expression of a gelatinase B promoter-lacZ fusion gene in transgenic mice." JOURNAL OF INVESTIGATIVE DERMATOLOGY, vol. 106, no. 4, 1996, page 906 XP000995143 Annual Meeting of the Society for Investigative Dermatology;Washington, D.C., USA; May 1-5, 1996 ISSN: 0022-202X
- IKAWA M ET AL: "Green fluorescent protein as a marker in transgenic mice" DEVELOPMENT GROWTH AND DIFFERENTIATION,JP,JAPANESE SOCIETY OF DEVELOPMENTAL BIOLOGISTS,, vol. 37, 1 August 1995 (1995-08-01), pages 455-459, XP002086829 ISSN: 0012-1592
- KEMP et al., "The Glycosylation of alpha-1-Antitrypsin in Transgenic Mice", ANIMAL CELL TECHNOLOGY: BASIC & APPLIED ASPECTS, 8TH ANNUAL CONFERENCE, Edited by FUNATSU et al., THE NETHERLANDS: KLUWER ACADEMIC PUBLISHERS, 1997, Vol. 8, pages 517-522, XP002910418
- DAMAK et al., "Expression of the Human Neutrophil Elastase Gene in the Lungs of Transgenic Mice", JOURNAL OF CELLULAR AND BIOCHEMISTRY SUPPLEMENT, 03 April 1992, Abstract V102, XP002910419
- SIMON et al., "Suppressor and Activator Functions Mediated by a Repeated Heptad Sequence in the Liver Fatty Acid-Binding Protein Gene (Fabpl)", J. OF BIOLOGICAL CHEMISTRY, 18 April 1997, Vol. 272, No. 16, pages 10652-10663, XP002910420
- YOSHIZAKI et al., "Increased Expression of Membrane Type 1-Matrix Metalloproteinase in Head and Neck Carcinoma", CANCER, 01 January 1997, Vol. 79, No. 1, pages 139-144, XP002910421
- RICE et al., "Detection of Gelatinase B Expression Reveals Osteoclastic Bone Resorption as a Feature of Early Calvarial Bone Development", BONE, December 1997, Vol. 21, No. 6, pages 479-486, XP002910422
- HIMELSTEIN et al., "Transcriptional Activation of the Matrix Metalloproteinase-9 Gene in an H-ras and V-myc Transformed Rat Embryo Cell Line", ONCOGENE, 1997, Vol. 14, No. 16, pages 1995-1998, XP002910423

### SEQUENCE LISTING

<110> The General Hospital Corporation
<120> Methods for evaluating a compound for its effect on skin
<130> epp82352
<140> 98918893.3
   <141> 1998-04-30
<150> us 60/069945
   <151> 1997-12-17
<160> 2
<170> Patent In version 3.0
<210> 1
   <211> 5063
   <212> DNA
   <213> artificial sequence
<220>
   <221> 220
   <222> (1)..(5063)
<220>
   <221> 223
   <222> (1)..(5063)
<400> 1
   tagttattac tagcgctacc ggactcagat ctcgagctca agcttcccga ggtcctgaag      60
   gaagagagta aagccatgtc tgctgttttc tagaggctgc tactgtcccc tttactgccc     120
   tgaagattca gcctgcggaa gacagggggt tgccccagtg gaattcccca gccttgccta     180
   gcagagccca ttccttccgc ccccagatga agcagggaga ggaagctgag tcaaagaagg     240
   ctgtcaggga gggaaaaaga ggacagagcc tggagtgtgg ggaggggttt ggggaggata     300
   tctgacctgg gagggggtgt tgcaaaaggc caaggatggg ccagggggat cattagtttc     360
   agaaagaagt ctcagggagt cttccatcac tttcccttgg ctgaccactg gaggctttca     420
   gaccaaggga tgggggatcc ctccagcttc atccccctcc ctccctttca tacagttccc 480
   acaagctctg cagtttgcaa aaccctaccc ctcccctgag ggcctgcggt ttcctgcggg 540
   tctggggtct tgcctgactt ggcagtggag actgcgggca gtggagagag gaggaggtgg 600
   tgtaagccct ttctcatgct ggtgctgcca cacacacaca cacacacaca cacacacaca 660
   cacacacaca ccctgacccc tgagtcagca cttgcctgtc aaggaggggt ggggtcacag 720
   gagcgcctcc ttaaagcccc cacaacagca gctgcagtca gacacctctg ccctcaccgg 780
   taccgcgggc ccgggatcca agatctcggt actcgaggaa ctaaaaaacc agaaagttaa 840
   ctggtaagtt tagtcttttt gtcttttatt tcaggtcccg gatccggtgg tggtgcaaat 900
   caaagaactg ctcctcagtg gatgttgcct ttacttctag gcctgtacgg aagtgttact 960
   tctgctctaa aagctgcgga attgtacccg cggccgcaat tcccggtcgc caccatggtg 1020
   agcaagggcg aggagctgtt caccggggtg gtgcccatcc tggtcgagct ggacggcgac 1080
   gtaaacggcc acaagttcag cgtgtccggc gagggcgagg gcgatgccac ctacggcaag 1140
   ctgaccctga agttcatctg caccaccggc aagctgcccg tgccctggcc caccctcgtg 1200
   accaccctga cctacggcgt gcagtgcttc agccgctacc ccgaccacat gaagcagcac 1260
   gacttcttca agtccgccat gcccgaaggc tacgtccagg agcgcaccat cttcttcaag 1320
   gacgacggca actacaagac ccgcgccgag gtgaagttcg agggcgacac cctggtgaac 1380
   cgcatcgagc tgaagggcat cgacttcaag gaggacggca acatcctggg gcacaagctg 1440
   gagtacaact acaacagcca caacgtctat atcatggccg acaagcagaa gaacggcatc 1500
   aaggtgaact tcaagatccg ccacaacatc gaggacggca gcgtgcagct cgccgaccac 1560
   taccagcaga acacccccat cggcgacggc cccgtgctgc tgcccgacaa ccactacctg 1620
   agcacccagt ccgccctgag caaagacccc aacgagaagc gcgatcacat ggtcctgctg 1680
   gagttcgtga ccgccgccgg gatcactctc ggcatggacg agctgtacaa gtaaagcggc 1740
   cgcgactcta gatcataatc agccatacca catttgtaga ggttttactt gctttaaaaa 1800
   acctcccaca cctccccctg aacctgaaac ataaaatgaa tgcaattgtt gttgttaact 1860
   tgtttattgc agcttataat ggttacaaat aaagcaatag catcacaaat ttcacaaata 1920
   aagcattttt ttcactgcat tctagttgtg gtttgtccaa actcatcaat gtatcttaag 1980
   gcgtaaattg taagcgttaa tattttgtta aaattcgcgt taatttttgt taaatcagct 2040
   catttttaac caataggccg aaatcggcaa aatcccttat aaatcaaaag aatagaccga 2100
   gatagggttg agtgttgttc cagtttggaa caagagtcca ctattaaaga acgtggactc 2160
   caacgtcaaa gggcgaaaaa ccgtctatca gggcgatggc ccactacgtg aaccatcacc 2220
   ctaatcaagt tttttggggt cgaggtgccg taaagcacta aatcggaacc ctaaagggag 2280
   cccccgattt agagcttgac ggggaaagcc ggcgaacgtg gcgagaaagg aagggaagaa 2340
   agcgaaagga gcgggcgcta gggcgctggc aagtgtagcg gtcacgctgc gcgtaaccac 2400
   cacacccgcc gcgcttaatg cgccgctaca gggcgcgtca ggtggcactt ttcggggaaa 2460
   tgtgcgcgga acccctattt gtttattttc taaatacatt caaatatgta tccgctcatg 2520
   agacaataac cctgataaat gcttcaataa tattgaaaaa ggaagagtcc tgaggcggaa 2580
   agaaccagct gtggaatgtg tgtcagttag ggtgtggaaa gtccccaggc tccccagcag 2640
   gcagaagtat gcaaagcatg catctcaatt agtcagcaac caggtgtgga aagtccccag 2700
   gctccccagc aggcagaagt atgcaaagca tgcatctcaa ttagtcagca accatagtcc 2760
   cgcccctaac tccgcccatc ccgcccctaa ctccgcccag ttccgcccat tctccgcccc 2820
   atggctgact aatttttttt atttgcagag gccgaggccg cctcggcctc tgagctattc 2880
   cagaagtagt gaggaggctt ttttggaggc ctaggctttt gcaaagatcg atcaagagac 2940
   aggatgagga tcgtttcgca tgattgaaca agatggattg cacgcaggtt ctccggccgc 3000
   ttgggtggag aggctattcg gctatgactg ggcacaacag acaatcggct gctctgatgc 3060
   cgccgtgttc cggctgtcag cgcaggggcg cccggttctt tttgtcaaga ccgacctgtc 3120
   cggtgccctg aatgaactgc aagacgaggc agcgcggcta tcgtggctgg ccacgacggg 3180
   cgttccttgc gcagctgtgc tcgacgttgt cactgaagcg ggaagggact ggctgctatt 3240
   gggcgaagtg ccggggcagg atctcctgtc atctcacctt gctcctgccg agaaagtatc 3300
   catcatggct gatgcaatgc ggcggctgca tacgcttgat ccggctacct gcccattcga 3360
   ccaccaagcg aaacatcgca tcgagcgagc acgtactcgg atggaagccg gtcttgtcga 3420
   tcaggatgat ctggacgaag agcatcaggg gctcgcgcca gccgaactgt tcgccaggct 3480
   caaggcgagc atgcccgacg gcgaggatct cgtcgtgacc catggcgatg cctgcttgcc 3540
   gaatatcatg gtggaaaatg gccgcttttc tggattcatc gactgtggcc ggctgggtgt 3600
   ggcggaccgc tatcaggaca tagcgttggc tacccgtgat attgctgaag agcttggcgg 3660
   cgaatgggct gaccgcttcc tcgtgcttta cggtatcgcc gctcccgatt cgcagcgcat 3720
   cgccttctat cgccttcttg acgagttctt ctgagcggga ctctggggtt cgaaatgacc 3780
   gaccaagcga cgcccaacct gccatcacga gatttcgatt ccaccgccgc cttctatgaa 3840
   aggttgggct tcggaatcgt tttccgggac gccggctgga tgatcctcca gcgcggggat 3900
   ctcatgctgg agttcttcgc ccaccctagg gggaggctaa ctgaaacacg gaaggagaca 3960
   ataccggaag gaacccgcgc tatgacggca ataaaaagac agaataaaac gcacggtgtt 4020
   gggtcgtttg ttcataaacg cggggttcgg tcccagggct ggcactctgt cgatacccca   4080
   ccgagacccc atggggccaa tacgcccgcg tttcttcctt ttccccaccc caccccccaa 4140
   gttcgggtga aggcccaggg ctcgcagcca acgtcggggc ggcaggccct gccatagcct 4200
   caggttactc atatatactt tagatttgat taaaacttca tttttaattt aaaaggatct 4260
   aggtgaagat cctttttgat aatctcatga ccaaaatccc ttaacgtgag ttttcgttcc 4320
   actgagcgtc agaccccgta gaaaagatca aaggatcttc ttgagatcct ttttttctgc 4380
   gcgtaatctg ctgcttgcaa acaaaaaaac caccgctacc agcggtggtt tgtttgccgg 4440
   atcaagagct accaactctt tttccgaagg taactggctt cagcagagcg cagataccaa 4500
   atactgtcct tctagtgtag ccgtagttag gccaccactt caagaactct gtagcaccgc 4560
   ctacatacct cgctctgcta atcctgttac cagtggctgc tgccagtggc gataagtcgt 4620
   gtcttaccgg gttggactca agacgatagt taccggataa ggcgcagcgg tcgggctgaa 4680
   cggggggttc gtgcacacag cccagcttgg agcgaacgac ctacaccgaa ctgagatacc 4740
   tacagcgtga gctatgagaa agcgccacgc ttcccgaagg gagaaaggcg gacaggtatc 4800
   cggtaagcgg cagggtcgga acaggagagc gcacgaggga gcttccaggg ggaaacgcct 4860
   ggtatcttta tagtcctgtc gggtttcgcc acctctgact tgagcgtcga tttttgtgat 4920
   gctcgtcagg ggggcggagc ctatggaaaa acgccagcaa cgcggccttt ttacggttcc 4980
   tggccttttg ctggcctttt gctcacatgt tctttcctgc gttatcccct gattctgtgg 5040
   ataaccgtat taccgccatg cat 5063
<210> 2
   <211> 7381
   <212> DNA
   <213> artificial sequence
<220>
   <221> 220
   <222> (1) .. (7381)
<220>
   <221> 223
   <222> (1) .. (7381)
<400> 2
   gaattcgcct gggtgaaagt gagttccccg ttggaggcaa cagacgagga gaggatggaa 60
   ggcctggccc ccaagaatga gccctgaggt tcagggagcg gctggagtga gccggcccca 120
   gatctcccga ggtcctgaag gaagagagta aagccatgtc tgctgttttc tagaggctgc 180
   tactgtcccc tttactgccc tgaagattca gcctgcggaa gacagggggt tgccccagtg 240
   gaattcccca gccttgccta gcagagccca ttccttccgc ccccagatga agcagggaga 300
   ggaagctgag tcaaagaagg ctgtcaggga gggaaaaaga ggacagagcc tggagtgtgg 360
   ggaggggttt ggggaggata tctgacctgg gagggggtgt tgcaaaaggc caaggatggg 420
   ccagggggat cattagtttc agaaagaagt ctcagggagt cttccatcac tttcccttgg 480
   ctgaccactg gaggctttca gaccaaggga tgggggatcc ctccagcttc atccccctcc 540
   ctccctttca tacagttccc acaagctctg cagtttgcaa aaccctaccc ctcccctgag 600
   ggcctgcggt ttcctgcggg tctggggtct tgcctgactt ggcagtggag actgcgggca 660
   gtggagagag gaggaggtgg tgtaagccct ttctcatgct ggtgctgcca cacacacaca 720
   cacacacaca cacacacaca cacacacaca ccctgacccc tgagtcagca cttgcctgtc 780
   aaggaggggt ggggtcacag gagcgcctcc ttaaagcccc cacaacagca gctgcagtca 840
   gacacctctg ccctcaccct cgaggaactg aaaaaccaga aagttaactg gtaagtttag 900
   tctttttgtc ttttatttca ggtcccggat ccggtggtgg tgcaaatcaa agaactgctc 960
   ctcagtggat gttgccttta cttctaggcc tgtacggaag tgttacttct gctctaaaag 1020
   ctgcggaatt gtacccgcgg ccgcaattcc cggggatcga aagagcctgc taaagcaaaa 1080
   aagaagtcac catgtcgttt actttgacca acaagaacgt gattttcgtt gccggtctgg 1140
   gaggcattgg tctggacacc agcaaggagc tgctcaagcg cgatcccgtc gttttacaac 1200
   gtcgtgactg ggaaaaccct ggcgttaccc aacttaatcg ccttgcagca catccccctt 1260
   tcgccagctg gcgtaatagc gaagaggccc gcaccgatcg cccttcccaa cagttgcgca 1320
   gcctgaatgg cgaatggcgc tttgcctggt ttccggcacc agaagcggtg ccggaaagct 1380
   ggctggagtg cgatcttcct gaggccgata ctgtcgtcgt cccctcaaac tggcagatgc 1440
   acggttacga tgcgcccatc tacaccaacg taacctatcc cattacggtc aatccgccgt 1500
   ttgttcccac ggagaatccg acgggttgtt actcgctcac atttaatgtt gatgaaagct 1560
   ggctacagga aggccagacg cgaattattt ttgatggcgt taactcggcg tttcatctgt 1620
   ggtgcaacgg gcgctgggtc ggttacggcc aggacagtcg tttgccgtct gaatttgacc 1680
   tgagcgcatt tttacgcgcc ggagaaaacc gcctcgcggt gatggtgctg cgttggagtg 1740
   acggcagtta tctggaagat caggatatgt ggcggatgag cggcattttc cgtgacgtct 1800
   cgttgctgca taaaccgact acacaaatca gcgatttcca tgttgccact cgctttaatg 1860
   atgatttcag ccgcgctgta ctggaggctg aagttcagat gtgcggcgag ttgcgtgact 1920
   acctacgggt aacagtttct ttatggcagg gtgaaacgca ggtcgccagc ggcaccgcgc 1980
   ctttcggcgg tgaaattatc gatgagcgtg gtggttatgc cgatcgcgtc acactacgtc 2040
   tgaacgtcaa aacccgaaac tgtggagcgc cgaaatcccg aatctctatc gtgcggtggt 2100
   tgaactgcac accgccgacg gcacgctgat tgaagcagaa gcctgcgatg tcggtttccg 2160
   cgaggtgcgg attgaaaatg gtctgctgct gctgaacggc aagccgttgc tgattcgagg 2220
   cgttaaccgt cacgagcatc atcctctgca tggtcaggtc atggatgagc agacgatggt 2280
   gcaggatatc ctgctgatga agcagaacaa ctttaacgcc gtgcgctgtt cgcattatcc 2340
   gaaccatccg ctgtggtaca cgctgtgcga ccgctacggc ctgtatgtgg tggatgaagc 2400
   caatattgaa acccacggca tggtgccaat gaatcgtctg accgatgatc cgcgctggct 2460
   accggcgatg agcgaacgcg taacgcgaat ggtgcagcgc gatcgtaatc acccgagtgt 2520
   gatcatctgg tcgctgggga atgaatcagg ccacggcgct aatcacgacg cgctgtatcg 2580
   ctggatcaaa tctgtcgatc cttcccgccc ggtgcagtat gaaggcggcg gagccgacac 2640
   cacggccacc gatattattt gcccgatgta cgcgcgcgtg gatgaagacc agcccttccc 2700
   ggctgtgccg aaatggtcca tcaaaaaatg gctttcgcta cctggagaga cgcgcccgct 2760
   gatcctttgc gaatacgccc acgcgatggg taacagtctt ggcggtttcg ctaaatactg 2820
   gcaggcgttt cgtcagtatc cccgtttaca gggcggcttc gtctgggact gggtggatca 2880
   gtcgctgatt aaatatgatg aaaacggcaa cccgtggtcg gcttacggcg gtgattttgg 2940
   cgatacgccg aacgatcgcc agttctgtat gaacggtctg gtctttgccg accgcacgcc 3000
   gcatccagcg ctgacggaag caaaacacca gcagcagttt ttccagttcc gtttatccgg 3060
   gcaaaccatc gaagtgacca gcgaatacct gttccgtcat agcgataacg agctcctgca 3120
   ctggatggtg gcgctggatg gtaagccgct ggcaagcggt gaagtgcctc tggatgtcgc 3180
   tccacaaggt aaacagttga ttgaactgcc tgaactaccg cagccggaga gcgccgggca 3240
   actctggctc acagtacgcg tagtgcaacc gaacgcgacc gcatggtcag aagccgggca 3300
   catcagcgcc tggcagcagt ggcgtctggc ggaaaacctc agtgtgacgc tccccgccgc 3360
   gtcccacgcc atcccgcatc tgaccaccag cgaaatggat ttttgcatcg agctgggtaa 3420
   taagcgttgg caatttaacc gccagtcagg ctttctttca cagatgtgga ttggcgataa 3480
   aaaacaactg ctgacgccgc tgcgcgatca gttcacccgt gcaccgctgg ataacgacat 3540
   tggcgtaagt gaagcgaccc gcattgaccc taacgcctgg gtcgaacgct ggaaggcggc 3600
   gggccattac caggccgaag cagcgttgtt gcagtgcacg gcagatacac ttgctgatgc 3660
   ggtgctgatt acgaccgctc acgcgtggca gcatcagggg aaaaccttat ttatcagccg 3720
   gaaaacctac cggattgatg gtagtggtca aatggcgatt accgttgatg ttgaagtggc 3780
   gagcgataca ccgcatccgg cgcggattgg cctgaactgc cagctggcgc aggtagcaga 3840
   gcgggtaaac tggctcggat tagggccgca agaaaactat cccgaccgcc ttactgccgc 3900
   ctgttttgac cgctgggatc tgccattgtc agacatgtat accccgtacg tcttcccgag 3960
   cgaaaacggt ctgcgctgcg ggacgcgcga attgaattat ggcccacacc agtggcgcgg 4020
   cgacttccag ttcaacatca gccgctacag tcaacagcaa ctgatggaaa ccagccatcg 4080
   ccatctgctg cacgcgaaga aggcacatgg ctgaatatcg acggtttcca tatggggatt 4140
   ggtggcgacg actcctggag cccgtcagta tcggcggaat tacagctgag cgccggtcgc 4200
   taccattacc agttggtctg gtgtcaaaaa taataataac cgggcaggcc atgtctgccc 4260
   gtatttcgcg taaggaaatc cattatgtac tatttaaaaa acacaaactt ttggatgttc 4320
   ggtttattct ttttctttta cttttttatc atgggagcct acttcccgtt tttcccgatt 4380
   tggctacatg acatcaacca tatcagcaaa agtgatacgg gtattatttt tgccgctatt 4440
   tctctgttct cgctattatt ccaaccgctg tttggtctgc tttctgacaa actcggcctc 4500
   gactctaggc ggccgcgggg atccagacat gataagatac attgatgagt ttggacaaac 4560
   cacaactaga atgcagtgaa aaaaatgctt tatttgtgaa atttgtgatg ctattgcttt 4620
   atttgtaacc attataagct gcaataaaca agttaacaac aacaattgca ttttcattat 4680
   gtttcaggtt cagggggagg tgtgggaggt tttttcggat cctctagagt cgacctgcag 4740
   gcatgcaagc ttggcgtaat catggtcata gctgtttcct gtgtgaaatt gttatccgct 4800
   cacaattcca cacaacatac gagccggaag cataaagtgt aaagcctggg gtgcctaatg 4860
   agtgagctaa ctcacattaa ttgcgttgcg ctcactgccc gctttccagt cgggaaacct 4920
   gtcgtgccag ctgcattaat gaatcggcca acgcgcgggg agaggcggtt tgcgtattgg 4980
   gcgctcttcc gcttccttcg ctcactgact cgctgcgctc ggtcgttcgg ctgcggcgag 5040
   cggtatcagc tcactcaaag gcggtaatac ggttatccac agaatcaggg gataacgcag 5100
   gaaagaacat gtgagcaaaa ggccagcaaa aggccaggaa ccgtaaaaag gccgcgttgc 5160
   tggcgttttt ccataggctc cgcccccctg acgagcatca caaaaatcga cgctcaagtc 5220
   agaggtggcg aaacccgaca ggactataaa gataccaggc gtttccccct ggaagctccc 5280
   tcgtgcgctc tcctgttccg accctgccgc ttaccggata cctgtccgcc tttctccctt 5340
   cgggaagcgt ggcgctttct catagctcac gctgtaggta tctcagttcg gtgtaggtcg 5400
   ttcgctccaa gctgggctgt gtgcacgaac cccccgttca gcccgaccgc tgcgccttat 5460
   ccggtaacta tcgtcttgag tccaacccgg taagacacga cttatcgcca ctggcagcag 5520
   ccactggtaa caggattagc agagcgaggt atgtaggcgg tgctacagag ttcttgaagt 5580
   ggtggcctaa ctacggctac actagaagga cagtatttgg tatctgcgct ctgctgaagc 5640
   cagttacctt cggaaaaaga gttggtagct cttgatccgg caaacaaacc accgctggta 5700
   gcggtggttt ttttgtttgc aagcagcaga ttacgcgcag aaaaaaagga tctcaagaag 5760
   atcctttgat cttttctacg gggtctgacg ctcagtggaa cgaaaactca cgttaaggga 5820
   ttttggtcat gagattatca aaaaggatct tcacctagat ccttttaaat taaaaatgaa 5880
   gttttaaatc aatctaaagt atatatgagt aaacttggtc tgacagttac caatgcttaa 5940
   tcagtgaggc acctatctca gcgatctgtc tatttcgttc atccatagtt gcctgactcc 6000
   ccgtcgtgta gataactacg atacgggagg gcttaccatc tggccccagt gctgcaatga 6060
   taccgcgaga cccacgctca ccggctccag atttatcagc aataaaccag ccagccggaa 6120
   gggccgagcg cagaagtggt cctgcacttt atccgcctcc atccagtcta ttaattgttg 6180
   ccgggaagct agagtaagta gtttcgccag ttaatagttg cgcaacgttg ttgccattgc 6240
   tacaggcatc gtggtgtcac gctcgtcgtt tggtatggct tcattcagct ccggttccca 6300
   acgatcaagg cgagttacat gatcccccat gttgtgcaaa aaagcggtta gctccttcgg 6360
   tcctccgatc gttgtcagaa gtaagttggc cgcagtgtta tcactcatgg ttatggcagc 6420
   actgcataat tctcttactg tcatgccatc cgtaagatgc ttttctgtga ctggtgagta 6480
   ctcaaccaag tcattctgag aatagtgtat gcggcgaccg agttgctctt gcccggcgtc 6540
   aatacgggat aataccgcgc cacatagcag aactttaaaa gtgctcatca ttggaaaacg 6600
   ttcttcgggg cgaaaactct caaggatctt accgctgttg agatccagtt cgatgtaacc 6660
   cactcgtgca cccaactgat cttcagcatc ttttactttc accagcgttt ctgggtgagc 6720
   aaaaacagga aggcaaaatg ccgcaaaaaa gggaataagg gcgacacgga aatgttgaat 6780
   actcatactc ttcctttttc aatattattg aagcatttat cagggttatt gtctcatgag 6840
   cggatacata tttgaatgta tttagaaaaa taaacaaata ggggttccgc gcacatttcc 6900
   ccgaaaagtg ccacctgacg tctaagaaac cattattatc atgacattaa cctataaaaa 6960
   taggcgtatc acgaggccct ttcgtctcgc gcgtttcggt gatgacggtg aaaacctctg 7020
   acacatgcag ctcccggaga cggtcacagc ttgtctgtaa gcggatgccg ggagcagaca 7080
   agcccgtcag ggcgcgtcag cgggtgttgg cgggtgtcgg ggctggctta actatgcggc 7140
   atcagagcag attgtactga gagtgcacca tatgcggtgt gaaataccgc acagatgcgt 7200
   aaggagaaaa taccgcatca ggcgccattc gccattcagg ctgcgcaact gttgggaagg 7260
   gcgatcggtg cgggcctctt cgctattacg ccagctggcg aaagggggat gtgctgcaag 7320
   gcgattaagt tgggtaacgc cagggttttc ccagtcacga cgttgtaaaa cgacggccag 7380
   t 7381

## Description

### BACKGROUND OF THE INVENTION

The invention relates to transgenic animals which express reporter genes coupled to promoters of genes involved in the health, aging, or appearance of the skin, e.g., the versican promoter, and methods of using such animals in evaluating treatments, e.g., compounds, for their effect on skin.

EP 0 633 315 describes a transgenic mouse which comprises the human K6 keratin promoter linked to a reporter gene, and suggests the use of this mouse for evaluating the effect of topical treatments on skin. Bernstein *et al.* (Journal of the American Academy of Dermatology, November 1997, pages 725-729) describe the evaluation of sunscreens with various sun protection factors in a transgenic mouse model in which the mouse comprises the human elastase promoter linked to a reporter gene.

### SUMMARY OF THE INVENTION

The inventors have discovered that transgenic animals having one or more constructs which include a skin-metabolism promoter coupled to a reporter gene can be used to evaluate a treatment, e.g., the removal of hair, e.g-, by plucking or shaving, or the administration of a compound for use in enhancing the health or appearance of the skin.

Accordingly the invention features a non-human transgenic animal having a reporter gene coupled to a versican promoter; and methods of evaluating treatments in accordance with any of claims 11, 12 or 13. We describe a method of evaluating a treatment, e.g., the removal of hair, e.g., by plucking or shaving, or the administration of a compound, for its effect on skin. The method includes:
providing a transgenic animal having a reporter gene coupled to a skin metabolism-related promoter, preferably a human reporter;
administering the treatment to the transgenic animal or a tissue therefrom; and
evaluating expression of the reporter gene, thereby evaluating the treatment for its effect on skin.

The treatment, e.g., the administration of a compound, can be administered to a live animal. In other embodiments, the treatment, e.g. the administration of a compound, is administered to a tissue, e.g., a cell taken from a transgenic animal.

The effect of the treatment, e.g., the administration of a compound, can be evaluated in a living transgenic animal, a dead transgenic animal, or tissue taken from either a living or transgenic mammal.

In preferred embodiments evaluating includes detection of a signal, e.g., a fluorescent signal, with a confocal microscope.

In preferred embodiments the evaluation of the expression of the reporter gene step is repeated at least once during the life of the animal. The first and a subsequent repetition of the step can be separated by as much as 1, 10, 30, 60, 90, 180, 365 or 700 days. Both the first and a subsequent repetition can be performed on a live animal, e.g. with the use of a confocal microscope.

In preferred embodiments, the treatment includes the administration of a compound and the compound is administered by: applying the compound to the skin of the transgenic animal; systemically administering the compound; orally administering the compound; or injecting the compound, preferably dermally or subcutaneously. In preferred embodiments, the compound is administered using a suitable delivery vehicle, for example, a surfactant or an agent which increases permeability in the skin, e.g., an SDS or DMSO containing formulation.

In preferred embodiments, the transgenic animal is a non-human transgenic animal. For example, the transgenic animal can be a transgenic mini-pig, a transgenic guinea-pig, a transgenic rat, or a transgenic mouse, e.g., a hairless mouse, a nude mouse, a senescence accelerated mouse, e.g., SAM mice, see Takeda et al., 1991, *L. Am. Geriatr.* 39:911-919, or a transgenic mutant mouse which exhibits a phenotype of accelerated aging. The most preferred animals are mice.

In preferred embodiments, the promoter is heterologous from the transgenic animal, i.e., the promoter is from another species. In other preferred embodiments the promoter is from the same species as the transgenic animal. In particularly preferred embodiments, the skin metabolism-related promoter is a human skin metabolism-related promoter.

In particularly preferred embodiments, the skin metabolism-related promoter is a versican promoter.

In preferred embodiments the reporter gene encodes a product which can be detected with relative ease, e.g., an enzyme, e.g., an enzyme which produces a colored or luminescent product In particularly preferred embodiments, the reporter gene can be a beta-galactosidase gene, a luciferase gene, a green fluorescent protein gene, an alkaline phosphatase gene, a horseradish peroxidase gene, or a chloramphenicol acetyl transferase gene.

In preferred embodiments, the treatment is administered repeatedly, prior to evaluation of reporter gene evaluation.

In preferred embodiments, the treatment includes the administration of a compound and the method further includes one or more subsequent administrations of the compound to the transgenic animal. In preferred embodiments, the compound is administered to the transgenic animal for a period of at least one, two, three, or four weeks. The compound can be administered at a constant level or at a range of different levels. In preferred embodiments, the compound is administered to the transgenic animal before, during, or after UV irradiation or other skin damaging treatment.

In preferred embodiments, the method further includes comparing the expression of the reporter gene to a control value, e.g., the level of expression of the reported gene in an untreated transgenic animal.

In preferred embodiments, the transgenic animal further includes a second reporter gene coupled to a second skin metabolism-related promoter, wherein the second skin metabolism-related promoter is different from the first skin metabolism-related promoter. The reporter gene coupled to the first promoter can be the same or different from the reporter gene coupled to the second promoter. For example, the transgenic animal can include: a first reporter gene coupled to the versican promoter and a second reporter gene coupled to the vascular endothelial growth factor promoter; a first reporter gene coupled to the versican promoter and a second reporter gene coupled to a hyaluronan synthase promoter. In preferred embodiments, the transgenic animal can include two constructs both of which are upregulated. In preferred embodiments, the transgenic animal can include two constructs both of which are downregulated.

In preferred embodiments, the method further includes evaluating the expression of the reporter gene coupled to the second skin metabolism-related promoter.

In preferred embodiments, the compound is: a cosmetic; a non-toxic substance; a substance approved for human drug or cosmetic use in one or more jurisdictions; a retinoid or derivative thereof; TGFβ; of TGFα.

In preferred embodiments, the method further includes administering a second treatment) to the transgenic animal. The second treatment can be one which injures or damages the skin, kills skin cells, or can include the removal of hair, e.g., by plucking, shaving, or application of a depilatory, or in general, induces an unwanted condition of the skin. The second treatment can be the application of water, a drying agent, an irritant, an inflammatory agent, light or UV irradiation. Reporter gene expression in response to the treatment can be determined in the presence of the second treatment, and optionally compared to the response seen in the absence of the second treatment.

In another aspect, the invention features, a method of evaluating a treatment, e.g., the removal of hair, e.g., by plucking or shaving, or the administration of a compound for its effect on skin. The method includes:
providing a transgenic animal, e.g., a mouse, having a reporter gene coupled to a, preferably human, versican promoter;
administering a treatment, e.g., the removal of hair, e.g., by plucking or shaving, or the administration of a compound, to the transgenic animal, or to a tissue taken therefrom; and
evaluating expression of the reporter gene, thereby evaluating the treatment for its effect on skin aging.

The treatment, e.g., the administration of a compound, can be administered to a live animal. In other embodiments the treatment, e.g., the administration of a compound, is administered to a tissue, e.g., a cell, taken from a transgenic animal.

The effect of the treatment, e.g., the administration of a compound, can be evaluated in a living transgenic animal, a dead transgenic animal, or tissue taken from either a living or dead transgenic animal.

In preferred embodiments evaluating includes detection of a signal, e.g., a fluorescent signal, with a confocal microscope.

In preferred embodiments the evaluation of the expression of the reporter gene step is repeated at least once during the life of the animal. The first and a subsequent repetition of the step can be separated by as much as 1, 10, 30, 60, 90, 180, 365, or 700 days. Both the first and a subsequent repetition can be performed on a live animal, e.g., with the use of a confocal microscope.

In preferred embodiments, the treatment includes the administration of a compound and the compound is administered by: applying the compound to the skin of the transgenic animal; systemically administering the compound; orally administering the compound; or injecting the compound, preferably dermally or subcutaneously. In preferred embodiments, the compound is administered using a suitable delivery vehicle, for example, a surfactant or an agent which increases permeability in the skin, e.g., an SDS or DMSO containing formulation.

In preferred embodiments, the transgenic animal is a non-human transgenic animal. For example, the transgenic animal can be a transgenic mini-pig, a transgenic guinea-pig, a transgenic rat, or a transgenic mouse, e.g., a hairless mouse, a nude mouse, a senescence accelerated mouse, e.g., SAM mice, see Takeda et al., 1991, *L. Am. Geriatr.* 39:911-919, or a transgenic mutant mouse which exhibits a phenotype of accelerated aging. The most preferred animals are mice.

In particularly preferred embodiments, the versican promoter is a human versican promoter.

In preferred embodiments the reporter gene encodes a product which can be detected with relative ease, e.g., an enzyme, e.g., an enzyme which produces a colored or luminescent product. In particularly preferred embodiments, the reporter gene can be a beta-galactosidase gene, a luciferase gene, a green fluorescent protein gene, an alkaline phosphatase gene, a horseradish peroxidase gene, or a chloramphenicol acetyl transferase gene.

In preferred embodiments the treatment is administered repeatedly, preferably prior to evaluation of reporter gene evaluation.

In preferred embodiments, the treatment includes the administration of a compound and the method further includes one or more subsequent administrations of the compound to the transgenic animal. In preferred embodiments, the compound is administered to the transgenic animal for a period of at least one, two, three, or four weeks. The compound can be administered at a constant level or at a range of different levels. In preferred embodiments, the compound is administered to the transgenic animal before, during, or after UV irradiation or other skin damaging treatment.

In preferred embodiments, the method further includes comparing the expression of the reporter gene to a control value, e.g., the level of expression of the reported gene in an untreated transgenic animal.

In preferred embodiments, the method further includes evaluating the expression of the reporter gene coupled to the second skin metabolism-related promoter.

In preferred embodiments, the compound is: a cosmetic; a non-toxic substance; a substance approved for human drug or cosmetic use in one or more jurisdictions; a retinoid or derivative thereof; TGFβ; of TGFα.

In preferred embodiments, the method further includes administering a second treatment to the transgenic animal. The second treatment can be one which injures or damages the skin, kills skin cells, or can include the removal of hair, e.g., by plucking, shaving, or application of a depilatory, or in general, induces an unwanted condition of the skin. The second treatment can be the application of water, a drying agent,-an irritant, an inflammatory agent, light or UV irradiation. Reporter gene expression in response to the treatment can be determined in the presence of the second treatment, and optionally compared to the response seen in the absence of the second treatment.

In preferred embodiments, the transgenic animal is a non-human transgenic animal. For example, the transgenic animal can be a transgenic mini-pig, a transgenic guinea-pig, a transgenic rat, or a transgenic mouse, e.g., a hairless mouse, a nude mouse, a senescence accelerated mouse, e.g., SAM mice, see Takeda et al., 1991, *L. Am. Geriatr.* 39:911-919, or a transgenic mutant mouse which exhibits a phenotype of accelerated aging. The most preferred animals are mice.

In particularly preferred embodiments, the matrix metalloproteinase promoter is a human matrix metalloproteinase promoter.

In preferred embodiments the reporter gene encodes a product which can be detected with relative ease, e.g., an enzyme, e.g., an enzyme which produces a colored or luminescent product. In particularly preferred embodiments, the reporter gene can be a beta-galactosidase gene, a luciferase gene, a green fluorescent protein gene, an alkaline phosphatase gene, a horseradish peroxidase gene, or a chloramphenicol acetyl transferase gene.

In preferred embodiments, the treatment is administered repeatedly, preferably prior to evaluation of reporter gene evaluation.

In preferred embodiments, the treatment includes the administration of a compound and the method further includes one or more subsequent administrations of the compound to the transgenic animal. In preferred embodiments, the compound is administered to the transgenic animal for a period of at least one, two, three, or four weeks. The compound can be administered at a constant level or at a range of different levels. In preferred embodiments, the compound is administered to the transgenic animal before, during, or after UV irradiation or other skin damaging treatment.

In preferred embodiments, the method further includes comparing the expression of the reporter gene to a control value, e.g., the level of expression of the reported gene in an untreated transgenic animal.

In preferred embodiments, the method further includes evaluating the expression of the reporter gene coupled to the second skin metabolism-related promoter.

In preferred embodiments, the compound is: a cosmetic; a non-toxic substance; a substance approved for human drug or cosmetic use in one or more jurisdictions; a retinoid or derivative thereof; TGFβ; of TGFα.

In preferred embodiments, the method further includes administering a second treatment to the transgenic animal. The second treatment can be one which injures or damages the skin, kills skin cells, or can include the removal of hair, e.g., by plucking, shaving, or application of a depilatory, or in general, induces an unwanted condition of the skin. The second treatment can be the application of water, a drying agent, an irritant, an inflammatory agent, light or UV irradiation. Reporter gene expression in response to the treatment can be determined in the presence of the second treatment, and optionally compared to the response seen in the absence of the second treatment.

In another aspect, the invention features, a method of evaluating a treatment, e.g., the removal of hair, e.g., by plucking or shaving, or the administration of a compound, for its effect on skin. The method includes:
providing a transgenic animal, e.g. a mouse, having a reporter gene coupled to a vascular endothelial growth factor promoter;
administering the treatment to the transgenic animal; and
evaluating expression of the reporter gene, thereby evaluating the treatment for its effect on skin aging.

The treatment, e.g., the administration of a compound, can be administered to a live animal. In other embodiments the treatment, e.g., the administration of a compound, is administered to a tissue, e.g., a cell, taken from a transgenic animal.

The effect of the treatment, e.g., the administration of a compound, can be evaluated in a living transgenic animal, a dead transgenic animal, or tissue taken from either a living or dead transgenic animal.

In preferred embodiments evaluating includes detection of a signal, e.g., a fluorescent signal, with a confocal microscope.

In preferred embodiments the evaluation of the expression of the reporter gene step is repeated at least once during the life of the animal. The first and a subsequent repetition of the step can be separated by as much as 1, 10, 30, 60, 90, 180, 365, or 700 days. Both the first and a subsequent repetition can be performed on a live animal, e.g., with the use of a confocal microscope.

In preferred embodiments, the treatment includes the administration of a compound and the compound is administered by: applying the compound to the skin of the transgenic animal; systemically administering the compound; orally administering the compound; or injecting the compound, preferably dermally or subcutaneously. In preferred embodiments, the compound is administered using a suitable delivery vehicle, for example, a surfactant or an agent which increases permeability in the skin, e.g., an SDS or DMSO containing formulation.

In preferred embodiments, the transgenic animal is a non-human transgenic animal. For example, the transgenic animal can be a transgenic mini-pig, a transgenic guinea-pig, a transgenic rat, or a transgenic mouse, e.g., a hairless mouse, a nude mouse, a senescence accelerated mouse, e.g., SAM mice, see Takeda et al., 1991, *L. Am. Geriatr.* 39:911-919, or a transgenic mutant mouse which exhibits a phenotype of accelerated aging. The most preferred animals are mice.

In particularly preferred embodiments, the vascular endothelial growth factor promoter is a human vascular endothelial growth factor promoter.

In preferred embodiments the reporter gene encodes a product which can be detected with relative ease, e.g., an enzyme, e.g., an enzyme which produces a colored or luminescent product. In particularly preferred embodiments, the reporter gene can be a beta-galactosidase gene, a luciferase gene, a green fluorescent protein gene, an alkaline phosphatase gene, a horseradish peroxidase gene, or a chloramphenicol acetyl transferase gene.

In preferred embodiments, the treatment is administered repeatedly, preferably prior to evaluation of reporter gene evaluation.

In preferred embodiments, the treatment includes the administration of a compound and the method further includes one or more subsequent administrations of the compound to the transgenic animal. In preferred embodiments, the compound is administered to the transgenic animal for a period of at least one, two, three, or four weeks. The compound can be administered at a constant level or at a range of different levels. In preferred embodiments, the compound is administered to the transgenic animal before, during, or after UV irradiation or other skin damaging treatment.

In preferred embodiments, the method further includes comparing the expression of the reporter gene to a control value, e.g., the level of expression of the reported gene in an untreated transgenic animal.

In preferred embodiments, the method further includes evaluating the expression of the reporter gene coupled to the second skin metabolism-related promoter.

In preferred embodiments, the compound is: a cosmetic; a non-toxic substance; a substance approved for human drug or cosmetic use in one or more jurisdictions; a retinoid or derivative thereof; TGFβ; of TGFα.

In preferred embodiments, the method further includes administering a second treatment to the transgenic animal. The second treatment can be one which injures or damages the skin, kills skin cells, or can include the removal of hair, e.g., by plucking, shaving, or application of a depilatory, or in general, induces an unwanted condition of the skin. The second treatment can be the application of water, a drying agent, an irritant, an inflammatory agent, light or UV irradiation. Reporter gene expression in response to the treatment can be determined in the presence of the second treatment, and optionally compared to the response seen in the absence of the second treatment.

In another aspect, the invention features, a method of evaluating a treatment, e.g., the removal of hair, e.g., by plucking or shaving, or the administration of a compound, for its effect on skin. The method includes:
providing a transgenic animal, e.g., a mouse, having a reporter gene coupled to a, preferably human, hyaluronan synthase promoter;
administering the treatment to the transgenic animal; and
evaluating expression of the reporter gene, thereby evaluating the treatment for its effect on skin aging.

The treatment, e.g., the administration of a compound, can be administered to a live animal. In other embodiments the treatment, e.g., the administration of a compound, is administered to a tissue, e.g., a cell, taken from a transgenic animal.

The effect of the treatment, e.g., the administration of a compound, can be evaluated in a living transgenic animal, a dead transgenic animal, or tissue taken from either a living or dead transgenic animal.

In preferred embodiments evaluating includes detection of a signal, e.g., a fluorescent signal, with a confocal microscope.

In preferred embodiments the evaluation of the expression of the reporter gene step is repeated at least once during the life of the animal. The first and a subsequent repetition of the step can be separated by as much as 1, 10, 30, 60, 90, 180, 365, or 700 days. Both the first and a subsequent repetition can be performed on a live animal, e.g., with the use of a confocal microscope.

In preferred embodiments, the treatment includes the administration of a compound and the compound is administered by: applying the compound to the skin of the transgenic animal; systemically administering the compound; orally administering the compound; or injecting the compound, preferably dermally or subcutaneously. In preferred embodiments, the compound is administered using a suitable delivery vehicle, for example, a surfactant or an agent which increases permeability in the skin, e.g., an SDS or DMSO containing formulation.

In preferred embodiments, the transgenic animal is a non-human transgenic animal. For example, the transgenic animal can be a transgenic mini-pig, a transgenic guinea-pig, a transgenic rat, or a transgenic mouse, e.g., a hairless mouse, a nude mouse, a senescence accelerated mouse, e.g., SAM mice, see Takeda et al., 1991, *L. Am. Geriatr.* 39:911-919, or a transgenic mutant mouse which exhibits a phenotype of accelerated aging. The most preferred animals are mice.

In particularly preferred embodiments, the hyaluronan synthase promoter is a human hyaluronan synthase promoter.

In preferred embodiments the reporter gene encodes a product which can be detected with relative ease, e.g., an enzyme, e.g., an enzyme which produces a colored or luminescent product. In particularly preferred embodiments, the reporter gene can be a beta-galactosidase gene, a luciferase gene, a green fluorescent protein gene, an alkaline phosphatase gene, a horseradish peroxidase gene, or a chloramphenicol acetyl transferase gene.

In preferred embodiments, the treatment is administered repeatedly, preferably prior to evaluation of reporter gene evaluation.

In preferred embodiments, the treatment includes the administration of a compound and the method further includes one or more subsequent administrations of the compound to the transgenic animal. In preferred embodiments, the compound is administered to the transgenic animal for a period of at least one, two, three, or four weeks. The compound can be administered at a constant level or at a range of different levels. In preferred embodiments, the compound is administered to the transgenic animal before, during, or after UV irradiation or other skin damaging treatment.

In preferred embodiments, the method further includes comparing the expression of the reporter gene to a control value, e.g., the level of expression of the reported gene in an untreated transgenic animal.

In preferred embodiments, the method further includes evaluating the expression of the reporter gene coupled to the second skin metabolism-related promoter.

In preferred embodiments, the compound is: a cosmetic; a non-toxic substance; a substance approved for human drug or cosmetic use in one or more jurisdictions; a retinoid or derivative thereof; TGFβ; of TGFα.

In preferred embodiments, the method further includes administering a second treatment to the transgenic animal. The second treatment can be one which injures or damages the skin, kills skin cells, or can include the removal of hair, e.g., by plucking, shaving, or application of a depilatory, or in general, induces an unwanted condition of the skin. The second treatment can be the application of water, a drying agent, an irritant, an inflammatory agent, light or UV irradiation. Reporter gene expression in response to the treatment can be determined in the presence of the second treatment, and optionally compared to the response seen in the absence of the second treatment.

In another aspect, the invention features, a method of evaluating a treatment, e.g., the removal of hair, e.g., by plucking or shaving, or the administration of a compound, for its effect on skin. The method includes:
providing a transgenic animal, e.g., a mouse, having a reporter gene coupled to, a preferably human, MMP2 or MMP9, preferably a MMP9, promoter;
administering the treatment to the transgenic animal, or to a tissue taken therefrom; and
evaluating expression of the reporter gene, thereby evaluating the treatment for its effect on skin aging.

The treatment, e.g., the administration of a compound, can be administered to a live animal. In other embodiments the treatment, e.g., the administration of a compound, is administered to a tissue, e.g., a cell, taken from a transgenic animal.

The effect of the treatment, e.g., the administration of a compound, can be evaluated in a living transgenic animal, a dead transgenic animal, or tissue taken from either a living or dead transgenic animal.

In preferred embodiments evaluating includes detection of a signal, e.g., a fluorescent signal, with a confocal microscope.

In preferred embodiments the evaluation of the expression of the reporter gene step is repeated at least once during the life of the animal. The first and a subsequent repetition of the step can be separated by as much as 1, 10, 30, 60, 90, 180, 365, or 700 days. Both the first and a subsequent repetition can be performed on a live animal, e.g., with the use of a confocal microscope.

In preferred embodiments, the treatment includes the administration of a compound and the compound is administered by: applying the compound to the skin of the transgenic animal; systemically administering the compound; orally administering the compound; or injecting the compound, preferably dermally or subcutaneously. In preferred embodiments, the compound is administered using a suitable delivery vehicle, for example, a surfactant or an agent which increases permeability in the skin, e.g., an SDS or DMSO containing formulation.

In preferred embodiments, the transgenic animal is a non-human transgenic animal. For example, the transgenic animal can be a transgenic mini-pig, a transgenic guinea-pig, a transgenic rat, or a transgenic mouse, e.g., a hairless mouse, a nude mouse, a senescence accelerated mouse, e.g., SAM mice, see Takeda et al., 1991, *L. Am. Geriatr.* 39:911-919, or a transgenic mutant mouse which exhibits a phenotype of accelerated aging. The most preferred animals are mice.

In particularly preferred embodiments, a MMP2 or MMP9, preferably a MMP9, promoter is a human MMP2 or MMP9, preferably a MMP9, promoter.

In preferred embodiments the reporter gene encodes a product which can be detected with relative ease, e.g., an enzyme, e.g., an enzyme which produces a colored or luminescent product. In particularly preferred embodiments, the reporter gene can be a beta-galactosidase gene, a luciferase gene, a green fluorescent protein gene, an alkaline phosphatase gene, a horseradish peroxidase gene, or a chloramphenicol acetyl transferase gene.

In preferred embodiments, the treatment is administered repeatedly, preferably prior to evaluation of reporter gene evaluation.

In preferred embodiments, the treatment includes the administration of a compound and the method further includes one or more subsequent administrations of the compound to the transgenic animal. In preferred embodiments, the compound is administered to the transgenic animal for a period of at least one, two, three, or four weeks. The compound can be administered at a constant level or at a range of different levels. In preferred embodiments, the compound is administered to the transgenic animal before, during, or after UV irradiation or other skin damaging treatment.

In preferred embodiments, the method further includes comparing the expression of the reporter gene to a control value, e.g., the level of expression of the reported gene in an untreated transgenic animal.

In preferred embodiments, the method further includes evaluating the expression of the reporter gene coupled to the second skin metabolism-related promoter.

In preferred embodiments, the compound is: a cosmetic; a non-toxic substance; a substance approved for human drug or cosmetic use in one or more jurisdictions; a retinoid or derivative thereof; TGFβ; of TGFα.

In preferred embodiments, the method further includes administering a second treatment to the transgenic animal. The second treatment can be one which injures or damages the skin, kills skin cells, or can include the removal of hair, e.g., by plucking, shaving, or application of a depilatory, or in general, induces an unwanted condition of the skin. The second treatment can be the application of water, a drying agent, an irritant, an inflammatory agent, light or UV irradiation. Reporter gene expression in response to the treatment can be determined in the presence of the second treatment, and optionally compared to the response seen in the absence of the second treatment.

In another aspect, the invention features, a method of evaluating a treatment, e.g., the removal of hair, e.g., by plucking or shaving, or the administration of a compound, for its effect on skin. The method includes:
providing a transgenic animal, e.g., a mouse, having a reporter gene coupled to a, preferably human, neutrophil elastase promoter;
administering the treatment to the transgenic animal, or to a tissue taken therefrom; and
evaluating expression of the reporter gene, thereby evaluating the treatment for its effect on skin aging.

The treatment, e.g., the administration of a compound, can be administered to a live animal. In other embodiments the treatment, e.g., the administration of a compound, is administered to a tissue, e.g., a cell, taken from a transgenic animal.

The effect of the treatment, e.g., the administration of a compound, can be evaluated in a living transgenic animal, a dead transgenic animal, or tissue taken from either a living or dead transgenic animal.

In preferred embodiments evaluating includes detection of a signal, e.g., a fluorescent signal, with a confocal microscope.

In preferred embodiments the evaluation of the expression of the reporter gene step is repeated at least once during the life of the animal. The first and a subsequent repetition of the step can be separated by as much as 1, 10, 30, 60, 90, 180, 365, or 700 days. Both the first and a subsequent repetition can be performed on a live animal, e.g., with the use of a confocal microscope.

In preferred embodiments, the treatment includes the administration of a compound and the compound is administered by: applying the compound to the skin of the transgenic animal; systemically administering the compound; orally administering the compound; or injecting the compound, preferably dermally or subcutaneously. In preferred embodiments, the compound is administered using a suitable delivery vehicle, for example, a surfactant or an agent which increases permeability in the skin, e.g., an SDS or DMSO containing formulation.

In preferred embodiments, the transgenic animal is a non-human transgenic animal. For example, the transgenic animal can be a transgenic mini-pig, a transgenic guinea-pig, a transgenic rat, or a transgenic mouse, e.g., a hairless mouse, a nude mouse, a senescence accelerated mouse, e.g., SAM mice, see Takeda et al., 1991, *L. Am. Geriatr.* 39:911-919, or a transgenic mutant mouse which exhibits a phenotype of accelerated aging. The most preferred animals are mice.

In particularly preferred embodiments, the neutrophil elastase promoter is a human neutrophil elastase promoter.

In preferred embodiments the reporter gene encodes a product which can be detected with relative ease, e.g., an enzyme, e.g., an enzyme which produces a colored or luminescent product. In particularly preferred embodiments, the reporter gene can be a beta-galactosidase gene, a luciferase gene, a green fluorescent protein gene, an alkaline phosphatase gene, a horseradish peroxidase gene, or a chloramphenicol acetyl transferase gene.

In preferred embodiments, the treatment is administered repeatedly, preferably prior to evaluation of reporter gene evaluation.

In preferred embodiments, the treatment includes the administration of a compound and the method further includes one or more subsequent administrations of the compound to the transgenic animal. In preferred embodiments, the compound is administered to the transgenic animal for a period of at least one, two, three, or four weeks. The compound can be administered at a constant level or at a range of different levels. In preferred embodiments, the compound is administered to the transgenic animal before, during, or after UV irradiation or other skin damaging treatment.

In preferred embodiments, the method further includes comparing the expression of the reporter gene to a control value, e.g., the level of expression of the reported gene in an untreated transgenic animal.

In preferred embodiments, the method further includes evaluating the expression of the reporter gene coupled to the second skin metabolism-related promoter.

In preferred embodiments, the compound is: a cosmetic; a non-toxic substance; a substance approved for human drug or cosmetic use in one or more jurisdictions; a retinoid or derivative thereof; TGFβ; of TGFα.

In preferred embodiments, the method further includes administering a treatment (other than the compound) to the transgenic animal. The treatment can be one which injures or damages the skin, kills skin cells, or in general, induces an unwanted condition of the skin. The treatment can be the application of water, a drying agent, an irritant, an inflammatory agent, light or UV irradiation. Reporter gene expression in response to the compound can be determined in the presence of the treatment, and optionally compared to the response seen in the absence of the treatment.

In another aspect, the invention features, a non-human transgenic animal described herein, e.g., a transgenic animal having a reporter gene coupled to a versican promoter.

In preferred embodiments, the transgenic animal is a non-human transgenic animal. For example, the transgenic animal can be a transgenic mini-pig, a transgenic guinea-pig, a transgenic rat, or a transgenic mouse, e.g., a hairless mouse, a nude mouse, a senescence accelerated mouse, e.g., SAM mice, see Takeda et al., 1991, *L. Am. Geriatr.* 39:911-919, or a transgenic mutant mouse which exhibits a phenotype of accelerated aging. The most preferred animals are mice.

In particularly preferred embodiments, the versican promoter is a human versican promoter.

In preferred embodiments the reporter gene encodes a product which can be detected with relative ease, e.g., an enzyme, e.g., an enzyme which produces a colored or luminescent product. In particularly preferred embodiments, the reporter gene can be a beta-galactosidase gene, a luciferase gene, a green fluorescent protein gene, an alkaline phosphatase gene, a horseradish peroxidase gene, or a chloramphenicol acetyl transferase gene.

In another aspect, the invention features, a non-human transgenic animal, e.g., a mouse, or a tissue taken therefrom, having a reporter gene coupled to a vascular endothelial growth factor promoter.

In preferred embodiments, the transgenic animal is a non-human transgenic animal. For example, the transgenic animal can be a transgenic mini-pig, a transgenic guinea-pig, a transgenic rat, or a transgenic mouse, e.g., a hairless mouse, a nude mouse, a senescence accelerated mouse, e.g., SAM mice, see Takeda et al., 1991, *L. Am. Geriatr.* 39:911-919, or a transgenic mutant mouse which exhibits a phenotype of accelerated aging. The most preferred animals are mice.

In particularly preferred embodiments, the vascular endothelial growth factor promoter is a human vascular endothelial growth factor promoter.

In preferred embodiments the reporter gene encodes a product which can be detected with relative ease, e.g.; an enzyme, e.g., an enzyme which produces a colored or luminescent product. In particularly preferred embodiments, the reporter gene can be a beta-galactosidase gene, a luciferase gene, a green fluorescent protein gene, an alkaline phosphatase gene, a horseradish peroxidase gene, or a chloramphenicol acetyl transferase gene.

In another aspect, the invention features, a non-human transgenic animal, e.g., a mouse, or a tissue taken therefrom, having a reporter gene coupled to a hyaluronan synthase promoter.

In preferred embodiments, the transgenic animal is a non-human transgenic animal. For example, the transgenic animal can be a transgenic mini-pig, a transgenic guinea-pig, a transgenic rat, or a transgenic mouse, e.g., a hairless mouse, a nude mouse, a senescence accelerated mouse, .g., SAM mice, see Takeda et al., 1991, *L. Am. Geriatr.* 39:911-919, or a transgenic mutant mouse which exhibits a phenotype of accelerated aging. The most preferred animals are mice.

In particularly preferred embodiments, the hyaluronan synthase promoter is a human hyaluronan synthase promoter.

In preferred embodiments the reporter gene encodes a product which can be detected with relative ease, e.g., an enzyme, e.g., an enzyme which produces a colored or luminescent product. In particularly preferred embodiments, the reporter gene can be a beta-galactosidase gene, a luciferase gene, a green fluorescent protein gene, an alkaline phosphatase gene, a horseradish peroxidase gene, or a chloramphenicol acetyl transferase gene.

In another aspect, the invention features, a non-human transgenic animal, e.g., a mouse, or a tissue taken therefrom, having a reporter gene coupled to a Type IV collagenase promoter.

In preferred embodiments, the transgenic animal is a non-human transgenic animal. For example, the transgenic animal can be a transgenic mini-pig, a transgenic guinea-pig, a transgenic rat, or a transgenic mouse, e.g., a hairless mouse, a nude mouse, a senescence accelerated mouse, e.g., SAM mice, see Takeda et al., 1991, *L. Am. Geriatr.* 39:911-919, or a transgenic mutant mouse which exhibits a phenotype of accelerated aging. The most preferred animals are mice.

In particularly preferred embodiments, a MMP2 or MMP9, preferably a MMP9, promoter is a human a MMP2 or MMP9, preferably a MMP9, promoter.

In preferred embodiments the reporter gene encodes a product which can be detected with relative ease, e.g., an enzyme, e.g., an enzyme which produces a colored or luminescent product. In particularly preferred embodiments, the reporter gene can be a beta-galactosidase gene, a luciferase gene, a green fluorescent protein gene, an alkaline phosphatase gene, a horseradish peroxidase gene, or a chloramphenicol acetyl transferase gene.

In another aspect, the invention features, a non-human transgenic animal, e.g., a mouse, or a tissue taken therefrom, having a reporter gene coupled to a neutrophil elastase promoter.

In preferred embodiments, the transgenic animal is a non-human transgenic animal. For example, the transgenic animal can be a transgenic mini-pig, a transgenic guinea-pig, a transgenic rat, or a transgenic mouse, e.g., a hairless mouse, a nude mouse, a senescence accelerated mouse, e.g., SAM mice, see Takeda et al., 1991, *L. Am. Geriatr.* 39:911-919, or a transgenic mutant mouse which exhibits a phenotype of accelerated aging. The most preferred animals are mice.

In particularly preferred embodiments, the neutrophil elastase promoter is a human neutrophil elastase promoter.

In preferred embodiments the reporter gene encodes a product which can be detected with relative ease, e.g., an enzyme, e.g., an enzyme which produces a colored or luminescent product. In particularly preferred embodiments, the reporter gene can be a beta-galactosidase gene, a luciferase gene, a green fluorescent protein gene, an alkaline phosphatase gene, a horseradish peroxidase gene, or a chloramphenicol acetyl transferase gene.

In another aspect, we also describe a promoter-reporter gene construct herein.

In another aspect, we also describe a method of analyzing GFP presence or distribution in a tissue. The method includes:
providing a tissue sample, e.g., a tissue section, which includes GFP;
evaluating or detecting fluorescent emission, or the lack of fluorescent emission, wherein said detecting step is performed prior to washing or fixing with an aqueous solution,
thereby analyzing GFP in a tissue.

In preferred embodiments the tissue is frozen prior to the detection step. The sample is not contacted with a fixing agent prior to detection.

In preferred embodiments: the tissue is from a transgenic animal, e.g., a transgenic mini-pig, guinea pig, rat or mouse, e.g., a hairless or nude mouse, a senescence accelerated mouse, e.g., SAM mice, see Takeda et al., 1991, *L. Am. Geriatr.* 39:911 -919, or a transgenic mutant mouse which exhibits a phenotype of accelerated aging; the GFP is expressed from a transgenic sequence encoding GFP or under the control of a transgenic control element, e.g., a transgenic promoter or enhancer.

In preferred embodiments the promoter is a human promoter.

In preferred embodiments detection includes examination of the sample with a microscope, e.g., a fluorescent or epi-fluorescent microscope.

In preferred embodiments evaluating includes detection of a signal, e.g., a fluorescent signal, with a confocal microscope.

Animals described herein can be used in this method.

In another aspect, we also describe a method of determining the stage of the hair cycle in an animal which expresses a reporter molecule in hair follicle, e.g., the outer root sheath of the hair follicle. The method includes evaluating or detecting the presence or absence of reporter expression, presence being associated with a growing hair cycle (anagen) and absence with a resting hair cycle (telegen and catogen).

In preferred embodiments the reporter molecule is under the control of a promoter expressed in the hair follicle, e,g., the outer root sheath.

In preferred embodiments the promoter is a human promoter.

In preferred embodiments the reporter molecule is GFP.

In preferred embodiments the animal is a transgenic animal, e.g.. a transgenic mini-pig, guinea pig, rat or mouse, e.g., a hairless or nude mouse, a senescence accelerated mouse. e.g., SAM mice, see Takeda et al., 1991, *L. Am. Geriatr.* 39:911-919, or a transgenic mutant mouse which exhibits a phenotype of accelerated aging;

Animals described herein can be used in this method.

In preferred embodiments the promoter is a VEGF promoter, a versican promoter, or other promoter described herein.

In another aspect, we also describe a method of analyzing wound healing. The method includes: providing an animal which expresses a reporter molecule under the control of a VEGF promoter, detecting the presence or absence of reporter molecule in a wound, thereby analyzing wound healing.

In preferred embodiments the detection step is reported.

In preferred embodiments, the animal, tissue from the animal, is subjected to a treatment, e.g., the administration of a compound. In such embodiments the method can be used to evaluate the effect of the treatment on wound healing. It may be desirable to compare results from a treated subject or tissue with an untreated subject or tissue.

In preferred embodiments the reporter molecule is GFP.

In preferred embodiments the animal is a transgenic animal, e.g.. a transgenic mini-pig, guinea pig, rat or mouse, e.g., a hairless or nude mouse, a senescence accelerated mouse. e.g., SAM mice, see Takeda et al., 1991, *L. Am. Geriatr.* 39:911-919, or a transgenic mutant mouse which exhibits a phenotype of accelerated aging.

Animals described herein can be used in this method.

In preferred embodiments the promoter is a human promoter.

In another aspect, we also describe a method of analyzing GFP expression in a transgenic animal having a GFP transgene. The method includes:
a first step of evaluating or detecting the presence or absence of GFP in the animal or in a tissue from the animal; and (optionally)
a second step of evaluating or detecting the presence or absence of GFP in the animal or in a tissue from the animal; and thereby analyzing GFP expression.

In preferred embodiments (in this method and in other methods disclosed herein) the GFP is red shifted GFP.

In preferred embodiments the animal is a transgenic animal, e.g., a transgenic mini-pig, guinea pig, rat or mouse, e.g., a hairless or nude mouse, a senescence accelerated mouse, e.g., SAM mice, see Takeda et al., 1991, *L. Am. Geriatr.* 39:911-919, or a transgenic mutant mouse which exhibits a phenotype of accelerated aging. An animal described herein can be used in this method.

In preferred embodiments at least 1, 5, 10, 20, 30, or 60, 180, 365 days elapse between first and second step.

In preferred embodiments the tissue is skin tissue.

In preferred embodiments the detection steps are performed on a live animal.

In preferred embodiments the promoter is a human promoter.

In preferred embodiments evaluating includes detection of a signal, e.g., a fluorescent signal, with a confocal microscope.

In another aspect, we describe a method of analyzing the expression of a transgene on a transgenic animal, e.g., a transgenic mouse or pig. The method includes:
providing a live transgenic animal;
evaluating or detecting the presence or absence of a reporter gene, e.g., GFP, encoded by a transgenic sequence or under the control of a transgenic control element, e.g., a promoter or enhancer;
thereby analyzing the expression of a transgene.

In preferred embodiments the animal is a transgenic animal, e.g., a transgenic mini-pig, guinea pig, rat or mouse, e.g., a hairless or nude mouse, a senescence accelerated mouse, e.g., SAM mice, see Takeda et al., 1991, *L. Am. Geriatr.* 39:911-919. or a transgenic mutant mouse which exhibits a phenotype of accelerated aging. Animals described herein can be used in the methods.

In preferred embodiments the reporter is under the control of a VEGF promoter or another promoter described herein.

In preferred embodiments the promoter is a human promoter.

In preferred embodiments the promoter is one which is expressed on the skin.

In preferred embodiments evaluating includes detection of a signal, e.g., a fluorescent signal, with a confocal microscope.

In preferred embodiments the tissue is skin tissue.

In preferred embodiments the detection steps are performed on a live animal.

In preferred embodiments the method is repeated at least once during the life of the animal. The first and a subsequent repetition of the method can be separated by as much as 1, 10, 30, 60, 90, 180, 365, or 700 days.

In another aspect, we describe a method of evaluating gene expression in a live animal. The method includes:
providing a live transgenic animal having a transgene which includes a reporter gene, e.g., GFP, e.g., red shifted GFP;
evaluating or detecting the presence or absence of reporter molecule, e.g., GFP, in the live transgenic animal, thereby evaluating gene expression in the live animal.

In preferred embodiments, the sequence which encodes a reporter gene is under the control of a preselected promoter, e.g., a human promoter. The preselected promoter can be, a skin metabolism-related promoter, e.g., : a promoter from a gene which encodes a transmembrane protein or a component of the extracellular matrix, such as a proteoglycan promoter, e.g., a versican promoter; a promoter from a protease expressed in the skin, e.g., a matrix metalloproteinase (MMP) promoter, e.g., an MMP1, MMP2, MMP3, MMP4, MMP5, MMP6, MMP7, MMP8, or MMP9 promoter; a promoter from a gene which affects vascular function, e.g., a vascular endothelial growth factor promoter; a hyaluronan synthase promoter, e.g., a hyaluronan synthase 1 promoter, a hyaluronan synthase 2 promoter, or a hyaluronan synthase 3 promoter; a promoter for a collagenase expressed in the skin, e.g., a MMP2 or MMP9, preferably a MMP9, promoter; or a neutrophil elastase promoter.

The VEGF promoter is a preferred promoter. In preferred embodiments the promoter is one which is up or down regulated in inflammatory angiogenesis, or neoplastic growth.

In preferred embodiments, the animal is a non-human transgenic animal. For example, the transgenic animal can be a transgenic mini-pig, a transgenic guinea-pig, a transgenic rat, or a transgenic mouse, e.g., a hairless mouse, a nude mouse, a senescence accelerated mouse, e.g., SAM mice, see Takeda et al., 1991, *L. Am. Geriatr.* 39:911-919, or a transgenic mutant mouse which exhibits a phenotype of accelerated aging. The most preferred animals are mice.

In preferred embodiments, a treatment is administered to the animal any of before, during, or after valuation of reporter gene expression. The treatment can be the administration of a compound. The compound can be administered by: applying the compound to the skin of the transgenic animal; systemically administering the compound; orally administering the compound; or injecting the compound, preferably dermally or subcutaneously. In preferred embodiments, the compound is administered using a suitable delivery vehicle, for example, a surfactant or an agent which increases permeability in the skin, e.g., an SDS or DMSO containing formulation.

In preferred embodiments evaluating includes detection of a signal, e.g., a fluorescent signal, with a confocal microscope.

In preferred embodiments evaluating includes detection of a signal. e.g., a fluorescent signal, with a confocal microscope.

In preferred embodiments the evaluation of the expression of the reporter gene step is repeated at least once during the life of the animal.- The first and a subsequent repetition of the step can be separated by as much as 1, 10. 30, 60, 90, 180, 365, or 700 days. Both the first and a subsequent repetition can be performed on a live animal, e.g., with the use of a confocal microscope.

Methods can be performed *in vivo,* with whole animals, or *in vitro,* that is, with tissue, e.g., skin, or cells, which are derived from a transgenic animal described herein or with cells, preferably skin cells or tissue, from cells transformed with a skin-metabolism promoter/reporter gene construct.

As used herein, a "transgenic animal" is an animal, e.g., a non-human mammal, e.g., a mini-pig, a guinea-pig, or a rodent, e.g., a mouse or a rat, in which one or more, and preferably essentially all, of the cells of the animal include a transgene. The transgene can be introduced into the cell, directly or indirectly by introduction into a precursor of the cell, e.g., by microinjection, transfection or infection, e.g.. by infection with a recombinant virus. The term genetic manipulation is directed to the introduction of a recombinant DNA molecule. This molecule may be integrated within a chromosome, or it may be extrachromosomally replicating DNA.

Transgenic animals can be, e.g., heterozygous or homozygous for a transgene.

As used herein, the term "rodent" refers to all members of the phylogenetic order *Rodentia.*

As used herein, the term "reporter gene" refers to a nucleic acid sequence which is fused downstream of a skin metabolism-related promoter, such that its expression is under the control of the promoter. Reporter genes usually encode a protein whose activity can be easily measured. For example, the reporter gene can be a gene encoding an assayable enzyme not found in the cell in nature, e.g., a beta-galactosidase gene, a luciferase gene, a green fluorescent protein gene, an alkaline phosphatase gene, a horseradish peroxidase gene, a chloramphenicol acetyl transferase gene, luciferase, and the like.

As used herein, the term "skin metabolism-related promoter" refers to a promoter which is transcriptionally active in the skin. It need not be skin-specific. The gene in which the promoter is naturally found can be a gene involved in the maintenance, or proper functioning of the skin. For example, the gene can be a gene encoding a protein which is part of the extracellular matrix, a protein involved in the maintenance or degradation of the extracellular matrix, or a protein involved in supplying nutrition to the skin.

As used herein, "administering a compound to an animal" refers to dispensing, delivering or applying a treatment to an animal or cell. Administration can be by topical administration, by parenteral or oral route, intramuscular injection, subcutaneous/intradermal injection, intravenous injection, buccal administration, transdermal delivery or administration by the intranasal or respiratory tract route. The most preferred administrations are topical application or subcutaneous or intradermal injection.

The methods of the invention allow rapid and efficient evaluation of compounds for their effect on skin.

Other features and advantages of the invention will be apparent from the following detailed description, and from the claims.

### DETAILED DESCRIPTION

The drawings are first briefly described.

### Brief Description of the Drawings

Figure 1 is a schematic representation of the human versican-Lac Z transgene construction.
Figure 2 is a schematic representation of the vascular endothelial growth factor-green fluorescent protein [VEGF-GFP] transgenic construct.

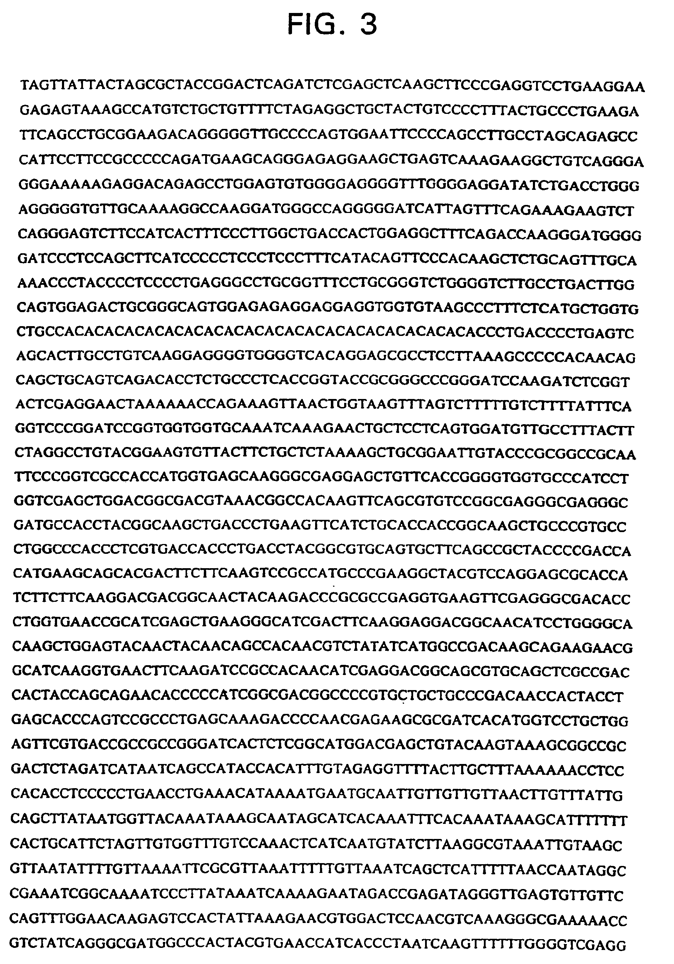

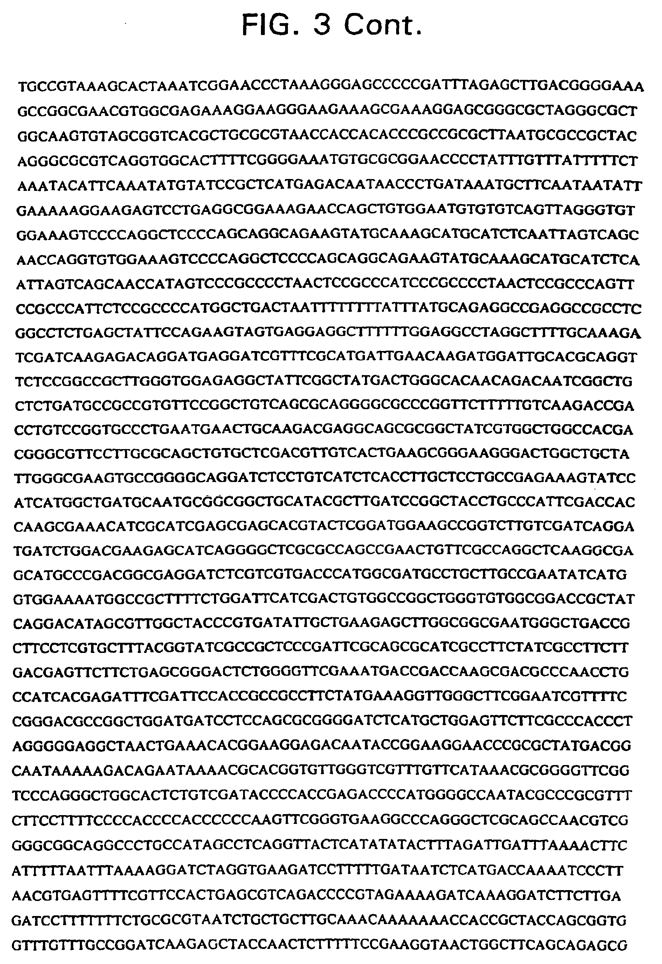

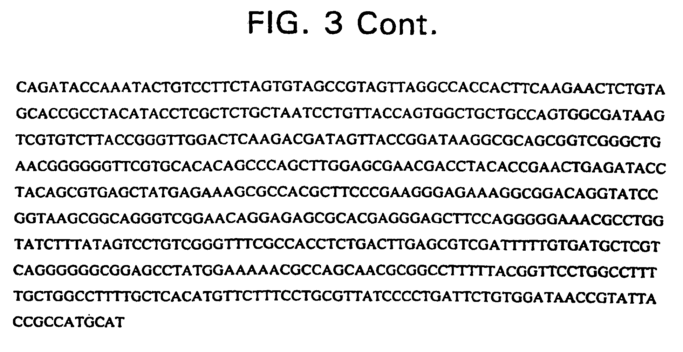

Figure 3 is a depiction of the nucleotide sequence of the 5069 pb MMP9 promoter-GFP construct.

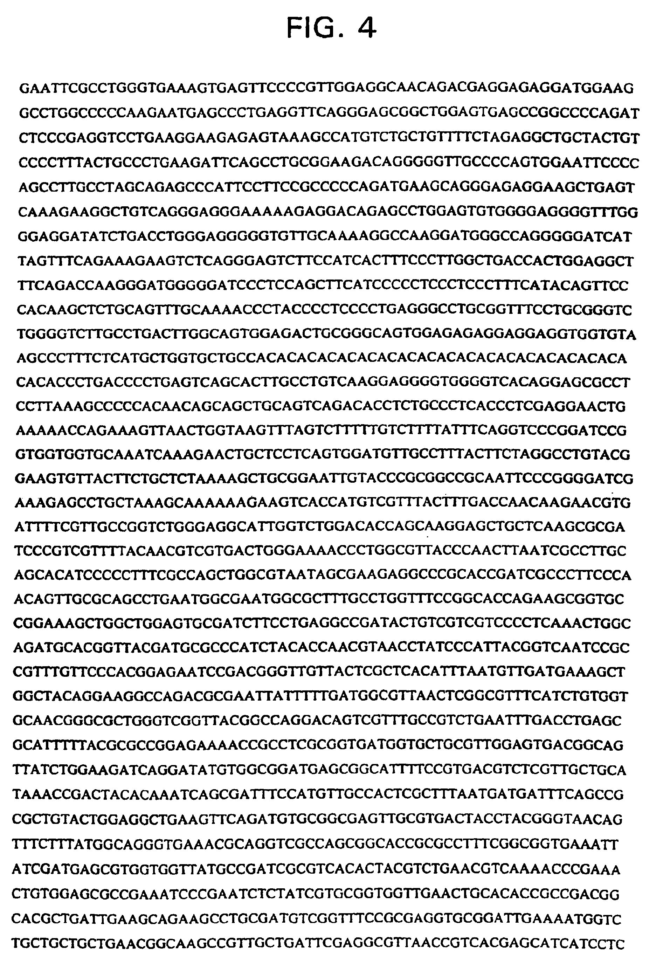

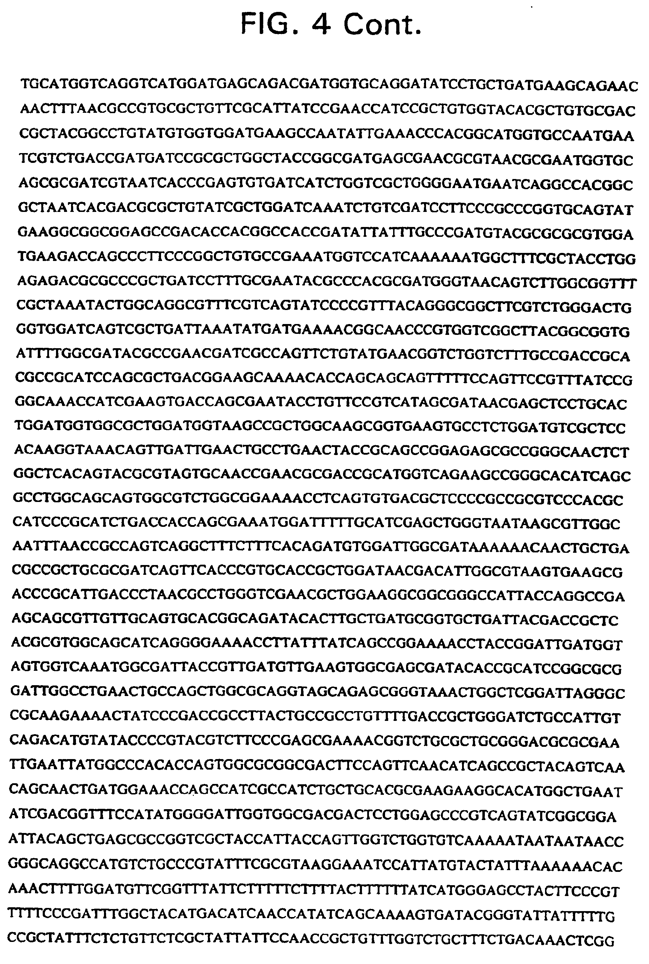

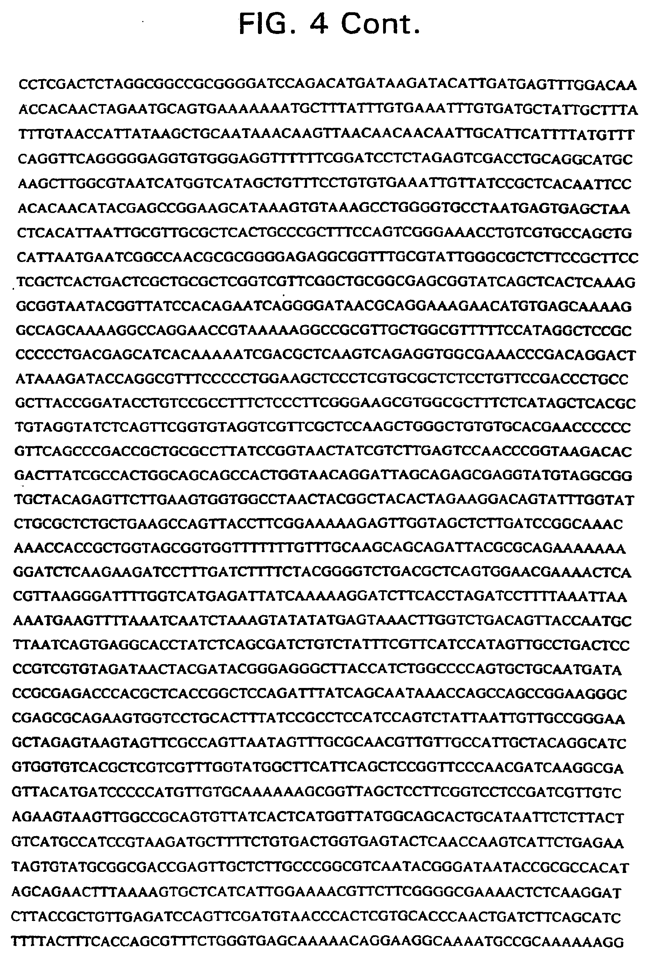

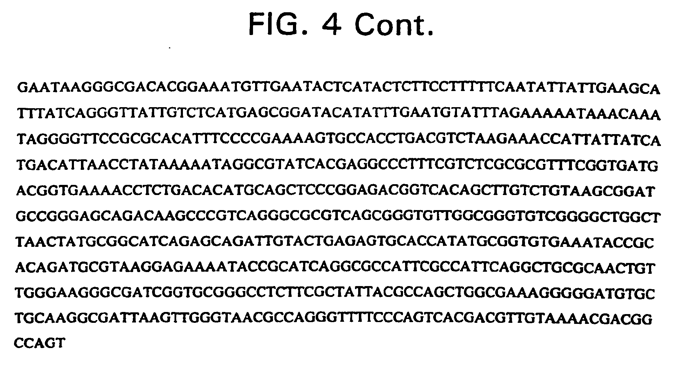

Figure 4 is a depiction of the nucleotide sequence of the 7383 pb MMP9 promoter-beta-Gal construct.

### Promoters

Methods of the invention allow evaluating a compound for its effect on the skin. Methods of the invention can be used to evaluate a compound for its effect on the health or appearance of the skin, e.g., for use as a cosmetic. The effect on skin is usually determined as an effect on the expression of a gene under the control of a skin-metabolism-related promoter. Such promoters include those which control the expression of: a product which is a component of the skin, e.g., the dermis or epidermis; a product which affects hydration or nutrition of the skin; a product which promotes the synthesis, or degradation, of components of the skin; a product which affects the vasculature of the skin; a product which affects hair follicle metabolism; a product which affects skin glandular structures; a product which affects subcutaneous musculature; a product which affects adipose tissue; or a product which affects cutaneous nerves.

Methods of the invention are useful for evaluating a compound for an effect on a parameter related to the appearance or health of the skin, for example, the elasticity of the skin, the propensity of the skin to wrinkle, the ability of the skin to retain fluids, e.g., water or an oil, the ability of the skin to resist or repair damage, e.g., light or UV induced damage, the metabolism of hair follicles including growth cycling or pigment deposition, subcutaneous muscle tone and function, or neurotransmission by cutaneous nerves. Generally, effects on these parameters will be evaluated indirectly, e.g., by the effect on the expression of a reporter gene under the control of a promoter which is normally coupled to a gene which encodes a product which affects any of the these parameters.

Examples of such skin-metabolism-related promoters include a versican promoter; a matrix metalloproteinase promoter; a vascular endothelial growth factor promoter; a hyaluronan synthase promoter; a MMP2 or MMP9, preferably a MMP9, promoter; and a neutrophil elastase promoter.

The versican promoter regulates the expression of the versican gene, described in Naso M. F. et al. (1994) *J. Biol. Chem.* 269(52): 32999-33008, the contents of which are incorporated herein by reference. Versican is a large modular chondroitin sulfate proteoglycan expressed in the dermis and the epidermis of the skin. Versican can bind large amounts of water while remaining attached to the extracellular matrix and can, therefore, hydrate and fill the skin. Accordingly, compounds which result in *upregulation* of this gene are preferred.

A matrix metalloproteinase promoter regulates the expression of a matrix metalloproteinase gene. The matrix metalloproteinases (MMPs) belong to a family of extracellular matrix proteases, described in Mauch C. et al. (1994) *Arch. Dermatol. Res.* 287:107-114. As the name implies, these matrix metalloproteinases are involved in the degradation of the extracellular matrix in, for example, the dermis and the epidermis of the skin. Accordingly, compounds which result in *downregulation* of this gene are preferred.

The vascular endothelial growth factor promoter regulates the expression of the vascular endothelial growth factor gene described in, for example, Tischer E. (1991) *J. Biol. Chem.* 266(18): 11947-11954, the contents of which are incorporated herein by reference. The vascular endothelial growth factor is a mitogen for vascular endothelial cells and, as a result, it can lead to proliferation of the microvasculature beneath the skin and increased vascular permeability. Increased microvasculature and vascular permeability allow for better nutrition (e.g., better nutrient delivery to the dermis and the epidermis) and hydration of the skin. Accordingly, compounds which result in *upregulation* of this gene are preferred.

The hyaluronan synthase (HAS 1-3) promoters regulate the expression of the hyaluronan synthase (HAS1-3) genes, described in Itano N. et al. (1996) *BBRC* 222: 816-820; Watanabe K. (1996) *J. Biol. Chem.* 271(38): 22945-22948; and Spicer A. P. (1997) *J. Biol. Chem.* 272(14): 8957-8961, respectively, the contents of which are incorporated herein by reference. Hyaluronan synthases, as the name implies, are enzymes involved in the synthesis of hyaluronan, a linear unbranched glycosaminoglycan. Hyaluronan binds versican and generally acts as an anchor for other proteoglycans, leading to the stabilization of the proteoglycan "network." As a result, hyaluronan (like versican) can assist in the hydration and filling of the skin. Accordingly, compounds which result in *upregulation* of this gene are preferred.

The type IV collagenase promoter regulates the expression of the type IV collagenase gene, described in Huhtala P. (1991) *J. Biol. Chem.* 266(25): 16485-16490, the contents of which are incorporated herein by reference. Type IV collagenase is another member of the extracellular matrix protease family and it is involved in the degradation of various components of the extracellular matrix in, for example, the dermis and the epidermis of the skin. Accordingly, compounds which result in *downgulation* of this gene are preferred.

The neutrophil elastase promoter regulates the expression of the neutrophil elastase gene, described in Takahashi H. (1988) *J. Biol. Chem.* 263(29): 14739-14747, the contents of which are incorporated herein by reference. Neutrophil elastase is a powerful serine protease capable of cleaving most protein components of the extracellular matrix (including elastin) in, for example, the dermis and the epidermis of the skin. Accordingly, compounds which result in *downgulation* of this gene are preferred.

### Transgenic animals

Transgenic animals which can be used in the methods of the invention include non-human mammals, such as pigs, e.g., mini-pigs, or guinea-pigs; or rodents, e.g., mice or rats, e.g., hairless mice (described in, for example, Begona M. et al. (1994) *Proc. Natl. Acad. Sci.* 91:7717-7721), nude mice, senescence accelerated mice (described in, for example, Takeda et al. (1991) *L. Am. Geriatr. Soc.* 39:911-19), or transgenic mutant mice which exhibit a phenotype of accelerated aging; in which one or more, and preferably essentially all, of the cells of the animal include a transgene. The transgenic animals can be homozygous or heterozygous for the transgene. Mice are a preferred subject animal.

### Construction of Transgenic Animals

Methods of making transgenic animals, e.g., mice, are known in the art. One approach is described below.

### Injection/Implantation of Embryos

Procedures for embryo manipulation and microinjection are described in, for example, *Manipulating the Mouse Embryo* (Cold Spring Harbor Laboratory Press, Cold Spring Harbor, NY., 1986, the contents of which are incorporated herein by reference). Mouse zygotes can be collected from six week old females that have been super ovulated with pregnant mares serum (PMS) followed 48 hours later with human chorionic gonadotropin. Primed females are placed with males and checked for vaginal plugs on the following morning. Pseudo pregnant females are selected for estrus, placed with proved sterile vasectomized males and used as recipients. Zygotes are collected and cumulus cells removed. Furthermore, blastocytes can be harvested. Pronuclear embryos are recovered from female mice mated to males. Females are treated with pregnant mare serum, PMS, to induce follicular growth and human chorionic gonadotropin, hCG, to induce ovulation. Embryos are recovered in a Dulbecco's modified phosphate buffered saline (DPBS) and maintained in Dulbecco's modified essential medium (DMEM) supplemented with 10% fetal bovine serum.

Microinjection of a transgenic construct can be performed using standard micro manipulators attached to a microscope. For instance, embryos are typically held in 100 microliter drops of DPBS under oil while being microinjected. DNA solution is microinjected into the male pronucleus. Successful injection is monitored by swelling of the pronucleus. Recombinant ES cells can be injected into blastocytes, using similar techniques. Immediately after injection embryos are transferred to recipient females, e.g. mature mice mated to vasectomized male mice. In a general protocol, recipient females are anesthetized, paralumbar incisions are made to expose the oviducts, and the embryos are transformed into the ampullary region of the oviducts. The body wall is sutured and the skin closed with wound clips.

### Screening for the Presence of the Targeting Construct

Transgenic animals can be identified after birth by standard protocols. DNA from tail tissue can be screened for the presence of the targeting construct using southern blots and/or PCR. Offspring that appear to be mosaics are then crossed to each other if they are believed to carry the targeting construct in their germ line to generate homozygous transgenic animals. If it is unclear whether the offspring will have germ line transmission, they can be crossed with a parental or other strain and the offspring screened for heterozygosity. The heterozygotes are identified by southern blots and/or PCR amplification of the DNA.

The heterozygotes can then be crossed with each other to generate homozygous transgenic offspring. Homozygotes may be identified by southern blotting of equivalent amounts of genomic DNA from mice that are the product of this cross, as well as mice that are known heterozygotes and wild type mice. Probes to screen the southern blots can be designed as set forth above.

Other means of identifying and characterizing the transgenic offspring are known in the art. For example, northern blots can be used to probe the mRNA for the presence or absence of transcripts encoding the reporter gene. In addition, western blots can be used to assess the level of expression of the transgene in various tissues of these offspring by probing the western blot with an antibody against the protein encoded by the transgene, or an antibody against the marker gene product, where this gene is expressed. Finally, *in situ* analysis (such as fixing the cells and labeling with antibody) and/or FACS (fluorescence activated cell sorting) analysis of various cells from the offspring can be performed using suitable antibodies to look for the presence or absence of the transgene product.

### Other Transgenic Animals

Other transgenic animals can be used in methods of the invention. Methods for the preparation of a variety of animals are known in the art. A protocol for the production of a transgenic pig can be found in White and Yannoutsos, *Current Topics in Complement Research: 64th* ***Forum in Immunology,* pp. 88-94;** US Patent No. 5,523,226; US Patent No. 5,573,933; PCT Application WO93/25071; and PCT Application WO95/04744. A protocol for the production of a transgenic rat can be found in Bader and Ganten, *Clinical and Experimental Pharmacology and Physiology,* Supp. 3:S81-S87, 1996. A protocol for the production of a transgenic cow can be found in *Transgenic Animal Technology, A Handbook,* 1994, ed., Carl A. Pinkert, Academic Press, Inc. A protocol for the production of a transgenic sheep can be found in *Transgenic Animal Technology, A Handbook,* 1994, ed., Carl A. Pinkert, Academic Press, Inc. All patents and references are incorporated herein by reference.

### Reporter Genes

The methods of the invention are based, at least in part, on coupling reporter genes to promoters of genes involved in skin metabolism. The reporter gene can be any gene which encodes a detectable product, preferably one which can be detected with relative ease, e.g., a gene product which is fluorescent, or which catalyzes a reaction which can be determined by formation of a colored product. For example, the reporter gene can encode an enzyme, e.g., an enzyme which produces a detectable product, e.g., a colored or a luminescent product. Reporter genes are known in the art and include a beta-galactosidase gene, a luciferase gene, a green fluorescent protein gene, an alkaline phosphatase gene, a horseradish peroxidase gene, or a chloramphenicol acetyl transferase gene. Reporter genes are described in, for example, Sambrook, J., Fritsh, E. F., and Maniatis, T. *Molecular Cloning: A Laboratory Manual. 2nd, ed, Cold Spring Harbor Laboratory,* Cold Spring Harbor Laboratory Press, Cold Spring Harbor, NY, 1989.

### EXAMPLES

### Example 1: Construction of the Transgene

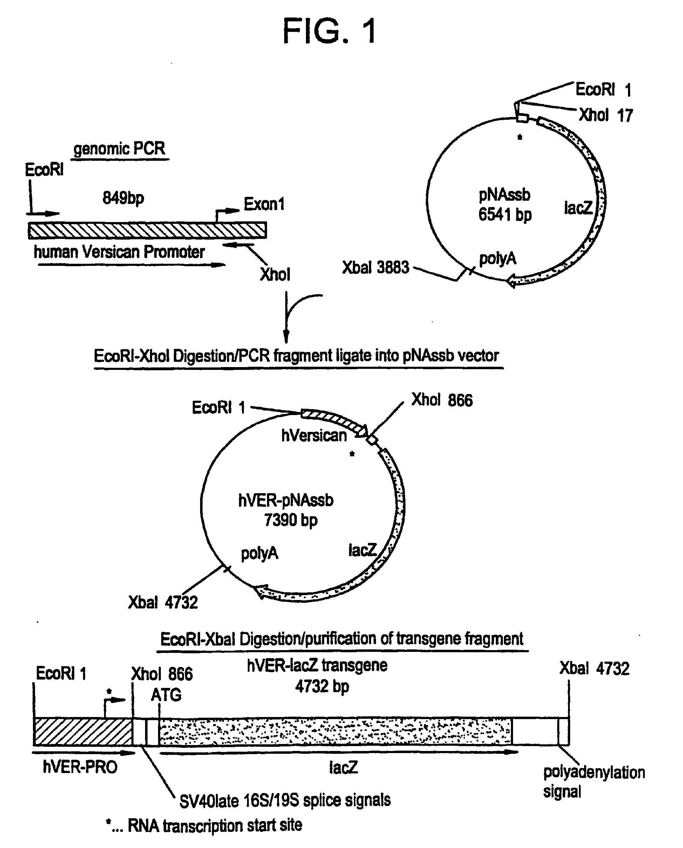

To construct the transgene, an 839 bp fragment of the human versican promoter (nucleotides -559 to +280; the transcription start site being +1) was obtained from human genomic DNA using art known PCR-based techniques. The versican promoter fragment was inserted into the pNASS2β vector (Clontech) which was linearized by restriction with *Xho*I*-EcoR*I as shown in Figure 1. This vector contains the β-galactosidase reporter gene (lacZ) as well as a polyadenylation signal. This vector also includes an RNA splice donor and acceptor sequence to optimize the chance of high level of transgene expression. To obtain the linearized transgene DNA fragment for pronuclear injection, the vector was cleaved with an *EcoR*I and an *Xba*I enzyme. The linearized transgene DNA fragment has a length of 4732 bp.

### Example 2: Generation of Transgenic Mice

The linearized transgene DNA fragment was injected into fertilized oocytes of DBA2 x C57BL6 (DBF1) mice (Charles River, Boston), and the eggs were implanted into pseudopregnant foster mothers. The offsprings (FO) were tested for chromosome integration of the human versican promoter fragment by southern blotting. Briefly, genomic DNA was isolated from the tails of 3-week old mice and digested with either the *BamH*I or the *Pst*I restriction endonuclease. The DNA fragments were separated on a gel and then transferred on a nylon membrane. The membranes were hybridized with a 1.3 BP *BamH*I*-EcoR*V fragment of the human versican transgene which was used to generate the transgenic mice. Seven independent lines showed transgene insertion by Southern analysis. The copy number of the transgene varied from one to more than ten.

### Example 3: β-galactosidase Histochemistry

Collected mouse embryos of varying stages (from E11.5d to E17.5d) of development were fixed with 0.5% glutaraldehyde in PBS for 30 minutes to 12 hours, depending on the embryo stage, transferred to 30% sucrose, and then frozen in OCT (Tissue Tek, CA) compound. 15µm-thick sections were prepared and loaded on slides. The sections were re-fixed in same fixative for 5 minutes and washed once with PBS, followed by a detergent wash solution for 10 minutes. Staining was performed in the reaction buffer containing 1 mg/ml X-gal (Sigma), 10 mM ferrocyanide, and 10 mM ferricyanide. Incubation was curried out for 3-6 hours at 37 °C. After washing with PBS, sections were either mounted or counterstained with eosin. For adult tissue, mice were perfused with the same fixative and the desired organs were dissected and post-fixed, sucrose submerged, and then frozen with OCT compound. For skin tissue, 5 mm strips of back skin tissue block were fixed for 15 minutes to an hour and stained with X-gal, as described above for 3-6 hours. After washing, the skin tissue strips were embedded in paraffin and cut in 8 µm sections using a microtome.

### Example 4: In situ hybridization

Radioactive *in situ* hybridization with 3' end labeled oligonucleotide probes was performed using methods known in the art. The specificity of the *in situ* signal was tested by hybridizing some sections with labeled oligonucleotides in the presence of excess unlabelled oligonucleotide.

The expression pattern of the transgene was examined by *in situ* hybridization of transgenic embryonic sections (E13.5) using lacZ and endogenous mouse versican probes. Using either probe, expression was observed in the developing limb bud, kidney, brain, and cartilage. LacZ expression was also examined by β-galactosidase histochemistry in E13.5, E15.5, E 17.5 embryos, through 7-day, 40-day newborns, and 4 month (adult) transgenic mice sections. Strong mesenchymal expression was observed in the kidney, brain, cartilage, and limb bud, as early as El3.Sd. The level of expression remained constant until birth. In 7-day old mice, the (β-galactosidase staining was decreased compared to embryonic tissue and almost no expression was observed in adult tissue. In contrast to other tissue, dermal papilla (anagen hair cycle) from 30-day old mice exhibited intense β-galactosidase staining again and continued to express the same level of β-galactosidase during the second hair cycle.

In skin tissue, hair bud (future dermal papilla in hair follicle) expressed strong β-galactosidase activity at E15.5d and continued to do so until 7 days after birth (during the first hair cycle). Occasional β-galactosidase staining in dermal fibroblasts was also observed in E15.5d -newborn skin. However, β-galactosidase staining was decreased in 7 day-old skin tissue.

### Example 5: UV irradiation

Irradiation with UVA was performed using a closely spaced array of five PUVA lamps. The energy output at 30 cm from array was measured with a UVA detector.

4-day old versican transgenic mice were irradiated on back skin with 30 J/cm2 UVA. Back skin tissue biopsy samples were processed 24 hours later for β-galactosidase staining and compared to untreated control skin from transgenic mice. β-galactosidase histochemistry showed increased β-galactosidase staining on the upper dermis of UVA irradiated skin compared to control.

### Example 6: VEGF-GFP Transgenic Mice

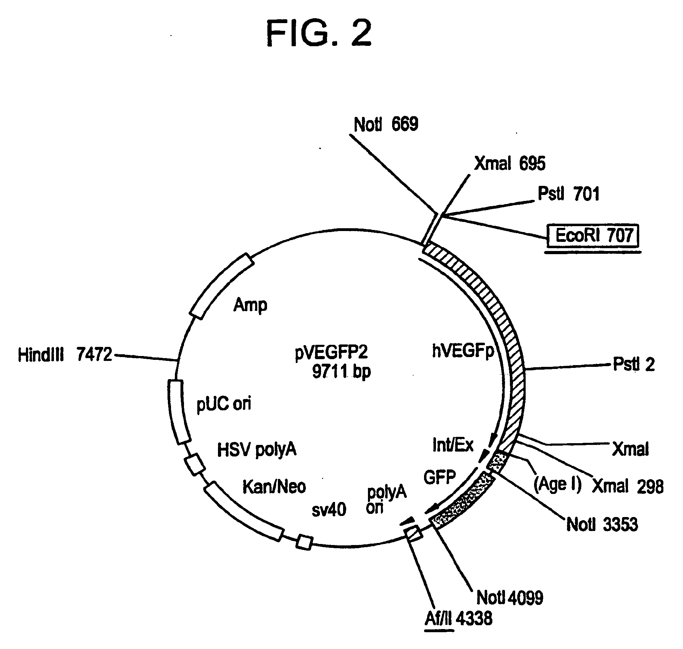

To elucidate the regulatory mechanisms of human VEGF gene expression *in vivo,* transgenic mice have been prepared which contain a green fluorescent protein reporter construct driven by a 3kbp fragment of the promoter region of human VEGF (shown in Figure 2). This construct was confirmed to be functional *in vitro* by transfection assays in cultured human keratinocytes. Three independent transgenic lines for VEGF-GFP were obtained and confirmed by PCR analysis. To examine constitutive endothelial expression, and keratinocyte inducible VEGF expression in wound skin, 2 mm skin punches were made in the back skin and biopsy samples were collected after 48 hours. Tissues were immediately fixed with 4% paraformaldehyde and cryostat sections were examined by fluorescent and confocal microscopy. VEGF-GFP transgenic lines exhibited bright GFP fluorescence in neomicrovascular networks beneath the epidermis and upper dermis, and occasionally in the deeper dermis. Hair follicles were also positive in some tissue. In wound tissue, continuous GFP expression was observed at the wound edge of the epidermis, but no keratinocyte expression was seen in non-wounded areas, confirming the inducibility of VEGF expression in healing wounds. Primary keratinocyte cultures prepared from the VEGF-GFP transgenic line also showed GFP fluorescence after 3 days in culture. Taken together these results indicate that GFP is a potentially useful tool to examine *in vitro* and *in vivo* VEGF expression. This work is described in more detail below.

Dynamic changes in the expression of green fluorescent protein driven by the human vascular endothelial growth factor promoter in transgenic mouse skin was analysed as follows..

Gene expression studies using transgenic mice most often utilize one of three classical reporter genes-lacZ (b-galactosidase) luciferase, or CAT (Chloramphenicol acetyl transferase). Detection of reporter gene activity usually requires the tissue to be removed from the animal for histochemical staining in the case of lacZ, or for complicated assays for luciferase and CAT, eliminating or at the least complicating the possibility of seeing a change in gene regulation in the living animal.

Recently an alternative fluorescent reporter molecule, green fluorescent protein (GFP), (Chalfee M, Tu Y, Euskirchen G, Ward WW and Prasher DC: Green fluorescent protein as a marker for gene expression. *Science* 263: 802-5, 1994) or modified GFP (EGFP), (Zhang G. Gurtu V and Kain SR: An enhanced green fluorescent protein allows sensitive detection of gene transfer in mammalian cells. *Biochem Biophys Res Commun* 227: 707-11, 1996) has been developed. Because GFP is intrinsically fluorescent, the signal is visible without treatment (Misteli T and Spector DL: Applications of the green fluorescent protein in cell biology and biotechnology. *Nat Biotechnol* 15: 961-4, 1997). When GFP is induced in the epidermis, it is possible to observe changes in gene expression without sacrificing the animals using confocal laser microscopy. The promoter of human vascular endothelial growth factor (VEGF), which is an angiogenic factor that induces *in vivo* angiogenesis and vascular permeability in malignant tumor tissue is used here to express GFP as a reporter gene in transgenic mouse skin. The expression of VEGF in epidermal keratinocytes was shown to be up-regulated at the edge of a healing epidermal wound, and also up-regulated topical application of phorbolesters, such as TPA.

The expression and responsiveness of GTP in signaling changes in gene activity of human VEGF-GFP in transgenic mice is described below. The expression patterns are consistent with that of endogenous VEGF, and show that GFP-derived fluorescent can be localized and visualized using confocal microscopy on intact tissue without any treatment following excisions.

### Transgene construct

Because PCR-based amplification of the GC-rish VEGF promoter is often difficult, a 5.0 kb fragment of VEGF genomic clone, which included the 5'-flanking DNA of human VEGF, was isolated from a human genomic library (Clontech, CA) using 500 bp cDNA fragment obtained by RT-PCR as a probe. The identified close was analyzed by restriction digestion, then a 2453 bp EcoRl-Agel fragment (-2271 to +91) (Tischer E, Mitchell R, Harman T, Silva M, Gospodarowicz D, Fiddes JC and Abraham JA: The human gene for vascular endothelial growth factor. Multiple protein forms are encoded through alternative exon splicing. *J Biol Chem* 266: 11947-54, 1991) was excised from the agarose gell. This promoter fragment was inserted into a GFP vector (pEGFP-1 cut EcoRI and Xmal). To maximize the efficiency of expression, 16s/19s splice donor and acceptor signals were also inserted between the promoter and GFP gene.

### In vitro expression of GFP vector.

The resultant transgene in the mammalian expression vector was first transfected into cultured human keratinocytes to confirm that the selected gene region promoted VEGF expression. Primary human keratinocytes were transfected with either the BEGF-GFP vector or a CMV promoter-driven GFP control vector (pEGFP-N1; Clontech). A porybrene and DMSO method was used for all transfections.

### Generation of Transgenic Mice

The linearized fragment for pronuclear injection was excised with EcoRI and AfIII, which also included a polyadenylation signal sequence. This transgene fragment was then injected into fertilized oocytes of DBA2xC57B16 (DBF1) mice (Charles River Laboratories, Boston, MA), and the eggs were implanted into pseudo pregnant foster mothers. The offspring (FO) were tested for chromosomal integration of the transgene by genome PCR. All experiments were done with F1 or F2 offspring mice.

For confocal microscopic observation of intact skin tissue, VEGF-GFP transgenic mice were generated in the *hairless* genetic background by breeding transgenic founder with SKH-1 *hairless* mice (Charles River Laboratories). Thus, male hemizygous transgenic mice (v/-HH) were mated with female *hairless* mice (-/-hh) to obtain the F2 generation of VEGF-GFP hairless mice (v/-hh), which could be easily selected by PCR detection and their *hairless* phenotype.

Tissue preparation for GFP detection and epimicroscopic observation solutions washed out or diffused GFP-derived fluorescence from the site of GFP expression. Ultimately, a freshly dissected, unfixed tissue block, was directly cut into 12 µm thick frozen sections using a cryostat. Epifluorescent microscopic observation was performed immediately after sectioning, without mounting medium or extended slide storage, using FITC/TRITC double excitation and an emission filter Chroma, *VT) to distinguish the GFP-derived fluorescent signal from tissue autofluorescence.

### Wounding and TPA treatment procedure

Skin wounds were produced with a 2 mm biopsy punch on the shaved back skin of young adult transgenic mice aged 4-6 weeks old. After 48 hours, normal and wounded tissues were collected and immediately frozen on dry ice.

For induction of VEGF mRNA by TPA, a single dose of 5 µg TPA in acetone was topically applied to back skin of transgenic mice and skin biopsy samples were collected after 12 hours.

All animal procedures had the approval of the MGH Animal Care and Use Committee.

### Anti-VEGF and anti-GFP immunohistochemistry

Cryostate sections of transgenic mice skin were fixed with 4% paraformaldehyde in PBS (pH 7.2) at room temperature for 5 min. Immunohistochemical staining was performed on slide mount sections using the ABC method as described by the manufacturer (Vector Labs. UK), and visualized by peroxidase reaction using DAB as a color substrate. Anti-GFP antiserum was purchased from Clontech.

### Confocal microscopy

For confocal microscopy observations, a coverslip was placed directly on the tissue. Sections or intact skin samples were analyzed using Leica TCS NT4D confocal microscope system with band pass filter 530/30nm, detecting emission at wavelengths between 515-545 nm. To visualize nuclei, sections were counterstained with 5µg/ml of 7-aminoactinomycin D (SIGMA, USA) solution for 20 min at ambient temperature, washed with PBS, and mounted with fluoromount-G (Southern Biotechnology, AL).

### VEGF promoter driven GFP expression in human keratinocytes

As the region of functional promoter activity for endogenous VEGF was not well characterized, 2.5 kb of 5'-flanking DNA of human VEGF was first inserted in a GFP mammalian vector and monitored in transfected primary human keratinocyte cultures. Control cultures were transfected with a CMV promoter-driven GFP vector, VEGF-GFP fluorescence was first visible at 48 hours after transfection, and only in a small percentage of the cells. CMV-GFP fluorescence was visible within 24 hours, and nearly all the cells were positive. The VEGF-GFP signal was similar in intensity to that of CMV-GFP control confirming that this construct is a functional in *in vitro* culture system. (Fluorescence in cultured keratinocytes transfected with human cytomegalovirus (CMV) immediate early promoter driven GFP as a positive control, and with VEGF-GFP, 48 hours after transfection were observed.)

### Expression of GFP in transgenic mice

In 30 founder mice, four lines positive for transgene chromosomal insertion were detected by PCR analysis. Three of these lines showed detectable GFP-derived fluorescent signal in the epidermis as assessed by epifluorescence microscopy of tail tissue, compared with negative wild type mice. GFP expression in new born transgenic mouse (Fluorescence was determined in fresh, unfixed tissue from the tail from a VEGF-GFP transgenic mouse and a similar tissue from a.negative wild-type litter mate. Fluorescence was also determined in tissues taken from new born VEGF-GFP transgenic mice: including lung (fluorescence seem in alveoli); lateral ventricle of the brain. (fluorescence was observed in lateral epithelium); and vertebral cartilage; (heart epithelium). The expression pattern of VEGF promoter was evaluated in F1 newborn pups of these three lines. The lung, kidney, and brain, previously been shown to express VEGF by in situ hybridization studies were chosen for examined for GFP fluorescence. As expected, GFP expression in lung alveoli and in the lateral ventricle wall in the brain was observed. It was difficult to detect a GFP signal in the flomerulus of kidney. GFP fluorescence was also detectable in the chondrocytes of developing cartilage tissue (e.g. vertebra cartilage).

### GFP is expressed at the edge of healing wounds

Since non-stimulated, normal epidermal keratinocytes showed only weak GFP fluorescence, we tested the inducibility of VEGF-driven GFP during wound healing. 48 hrs after removal of a skin biopsy, the healing wound epidermis beneath the scab showed a very strong and distinct fluorescent signal compared with adjacent unwounded epidermis. This fluorescent signal correlated with VEGF/GFP expression detected by anti-GFP and anti-VEGF immunohistochemistry in adjacent sections. GFP expression induced by skin wound healing. (VEGF-GFP transgenic mice were wounded by punch biopsy. Tissue from the wound was taken for evaluation by fluorescence microscopy. (a) In healing wound edge 48 hours after biopsy strong fluorescence was observed in the epithelium underlying the scab. (b) An adjacent section was reacted with anti-GFP antibodies. (c) An adjacent section was immunostained with antimouse VEGF antibodies. A control section was treated with normal goat serum. Non-specific peroxidase staining observed in the scabs as also seen in b and c.)

### Induction of GFP by TPA treatment

Induction of epidermal GFP by phorbolester application was also examined. Accumulation of fluorescent signal was observed 12 hours after TPA was applied to transgenic skin, whereas acetone alone showed only basal levels of GFP expression. This signal seemed to localize to the basoplateral surface of the epithelium rather than in either the keratinocyt cytosol or nucleus. Anti-GFP immunostaining produced a signal in the basal cell cytosol, indicating that GFP originated in basal cells, and at least part of the GFP protein remained within the cell.

### Direct detection of GFP by confocal microscopy

In order to localize the GFP fluorescence signal more precisely, fresh cryostat sections were fixed and incubated with 7-AAD for nuclear counter staining and examined under laser confocal microscope. As expected, most diffusable GFP was lost during this procedure, although wound healing-induced-epidermal expression remained unaffected. Occasional fluorescence was detectable in the unwounded epidermis, where the fluorescence appeared to localize outside basal cells as we observed. Even after fixation and intense washing, GFP-derived fluorescence in the outer root sheath of hair follicle remained unchanged. At higher magnification of the hair follicles, it can be seen that the fluorescent GFP signal completely co-localized with a nuclear maker, indicating that hair follicle GFP remains stably within the nucleus. This was also the case for the chonodrocytes in the vertebrae cartilage.

### Direct detection of GFP in the epidermis of intact skin by confocal microscopy

To test the possibility that GFP might be detected in the skin of a living GFP transgenic mouse, dissected *hairless* skin was directly examined by confocal imaging. Horizontal optical sections of the living epidermis revealed bright fluorescence. The equivalent cryostate section showed that this fluorescence is derived from the basal cells. Higher magnification revealed that GFP was localized in the extracellular space around the keratinocytes. X-Z confocal imaging confirmed that this signal was within the basal layer of the epidermis. Detection of GFP expression in intact skin by confocal microscopy. Fress unfixed transgenic mouse skin was biopsied, and immediately evaluated by scanning laser confocal microscopy. Horizontal optical sections of the epidermis show fluorescence surrounding individual epithelial cells. Cryostate sections of an equivalent skin region counterstained with 7-AAD to indicate nuclei, showed fluorescence at the dermal-epidermal junction. The confocal X-Z image indicates the fluorescence near the basal keratinocytes.

This work shows that the expression pattern of GFP driven by a human VEGF promotor is spatially and temporally equivalent to that seen with mRNA detection.

Pups from breedings of transgenic mice expressing GFP in the skin epidermis were easily selected by simple epifluorescent microscopy, without the more tedious procedures of southern blotting or genomic PCR.

There was initial difficult in detecting GFP-derived fluorescence in sections, even though fresh untreated tissue blocks emitted bright fluorescence. After testing a variety of procedures, it was found that aqueous solution treatment immediately washed out the GFP flourescence. This was partially overcome by fixing the tissue block before cryostate sectioning, though fluorescence from some sites, such as that initially present in epidermal keratinocytes, was still lost or its staining pattern was altered. Fixation induced fluorescence indistinguishable from that produced by GFP in the skin microvasculature. Fluorescence at this site was not seen in unfixed tissue, and VEGF expression has not been reported in the microvasculature in studies using *in situ* hybridization methods. Thus, the most efficient method to preserve the original GFP signal is to immediately cryostat section freshly dissected skin without OCT compound, and then promptly examine it by fluorescence microscopy. This unexpected difficulty may explain why, until now, there have been so few reports of successful GFP transgenic mice.

Prominent VEGF expression has been reported in lung, and in the lateral ventricle of the brain by *in situ* hybridization. Evaluation of the VRGF-GFP mice tissues confirm these findings. Failure of the kidney glomeruli to fluoresce may be due to a lack of tissue specific response element to 2.2 kb of 5'-flanking region of VEGF DNA used here for generating these transgenic mice, or to a signal below the detection threshold.

In skin from a 1 week old pup, normal epidermal keratinocyte showed basal levels of GFP expression, while bright fluorescence was observed in the outer root sheath of hair follicle. The skin of older mice did not show equivalent GFP expression in hair follicles, therefore, VEGF expression in the follicles may be hair cycle-dependent. Fluorescence in hair follicle and in chondrocytes, as seen in the vertebra and previously reported was particularly impressive. These signals were even stronger than those observed in lung or brain. Laser confocal observations revealed that GFP at these locations is confined within the nuclei. This nuclear localization may intensify the apparent signal since nuclear localization is likely to reduce the diffusion of the fluorescent product during tissue processing. The reason for localization to the nucleas by some tissues and not by others remains unexplained, but suggests that inclusion of a nuclear localization conseneus sequence with in the transgene might be useful.

The inducibility of GFP protein during wound healing model and after TPA treatment is shown herein, confirming that the reporter GFP expression pattern is highly VEGF promoter-dependent. Also, these data eliminated concerns about the possible delay in fluorescent signal production due to slow chromophore formation as reported in Drosophila embryos. (Davis I, Girdham CH, O'Farrell PH "A nuclear GFP that marks nuclei in living Drosophila embryos; maternal supply overcomes a delay in the appearance of zygotic fluorescence" *Dev. Biol.* 170:726-9 (1995)). In anticipation of this difficulty the red-shifted variant of GFP was selected for these studies, and the correct folding of the chromophore appears to occur in skin as well as in more internal tissues. There was also concern that GFP might have a prolonged half life in skin interfering with the ability to use the reporter to monitor long-term gene expression. The disappearance of fluorescence in hair follicles of older transgenic mouse skin indicates that GFP protein has a reasonable half life.

Laser confocal microscopic evaluation of intact skin for detection of the GFP signal indicates that GFP is readily detected within the epidermis. These results suggest the potential use of GFP transgenic mice for non-invasive monitoring of long-term gene expression *in vivo.* The technology for microscopic evaluation of living mouse skin is already in use. When combined with use of a transgenic GFP reporter gene, the system has a great advantage over conventional transgenic reporter gene animals methods because it reduces the number of animals used, utilizes a simple monitoring system, and allows long term monitoring of changes in gene expression on the same individual. Our successful *in vivo* expression of GFP in transgenic mouse skin should facilitate the understanding of VEGF gene expression in skin, by providing a useful monitoring and screening tool. Its use would be particularly attractive when bred to a mouse with a phenotype thought to involve VEGF expression. This model would also be advantageous in research focused on skin homeostasis during the aging process.

### Example 7: Developmental and Age-Related Changes of Human Versican Promoter Activity in Transgenic Mice Skin and Hair Dermal Papilla

To investigate further for temporal-spacious expression pattern for human versican, particularly in skin tissue, transgenic mice were generated containing a lacZ reporter construct driven by the functional promoter for the core protein of human versican and tissue sections from resultant transgenic mice ranged from embryos, newborn to adult stage were examined by b-galactosidase histochemistry.

### Construction of transgene

The 839 bp fragment of functional human versican promoter (from -559 to +280 according to the transcription start site as +1) including exon 1; (Naso MF, Zimmermann DR and Iozzo RV, "Characterization of the complete genomic structure of the human versican gene and functional analysis of its promoter", *J. Biol*. *Chem.* 269:32999-3008 (1994)) was obtained from human genomic DNA by PCR based technique and correct sequences were confirmed by direct sequencing. pNASS2β vector (Clontech) was used as a source of β-galactosidase reporter gene (lacZ) with polyadenylation signal. This vector also included RNA splice donor and acceptor sequence to optimize the chance of high level of transgene expression. Versican promoter fragment was inserted in front of splice donor/acceptor sequence using XhoI - EcoRI restriction site. Linearised transgene DNA for pronuclear injection was obtained as EcoRI-Xbal 4732bp fragment.

### Generation of Transgenic Mice

The linearized construct was injected into fertilized oocytes of DBA2xC57BL6(DBF1) mice (Charles River, Boston), and the eggs were implanted into pseudo pregnant foster mothers. The offspring(FO) were tested for the chromosome integration of the human versican promoter construct by Southern hybridization. Thus, genomic DNA was isolated from the tails of the mice at 3 weeks old and digested either BamHI or PstI restriction endonuclease. The fractionated DNA fragment on the nylon membranes were hybridized with 1.3kbp BamHI-EcoRV fragment of human versican transgene were used to generate transgenic mice. Seven independent lines which show transgene insertion by Southern analysis with different copy number from single insertion to more than ten.

### b-galactosidase histochemistry

Collected mouse embryos of early to mid stages (from E11.5d to E15.5d) of development were fixed with 0.5% glutaraldehyde in PBS for 30 min to 12 hrs depends on the stage and transferred to 30% sucrose, then frozen in OCT compound. Sections were cut 15um thickness and loaded on slides. Sections were re-fixed in same fixative for 5 min and was once with PBS, followed by detergent wash solution for 10 min. Staining reaction was done in the reaction buffer containing 1 mg/ml X-gal (Sigma), 10mM ferrocyanide, and 10mM ferricyanide. Incubation was curried out for 3-6 hrs at 37°C. After washing with PBS, sections were either mounted or counterstained with eosin. For adult tissue, mice were perfused with same fixative and desired organs were dissected and post-fixed, sucrose submerged, then frozen with OCT compound. In order to preserve better morphology some early to mid embryo (from E11.5d to E15.5d) were processed for paraffin section. For skin tissue, 5mm strip of back skin tissue block were fixed for 15 min-1 hr and proceed X-gal staining for 3-6 hrs. After wash, blocks were processed for paraffin embedding and cut 8 um section with microtome.

### In situ hybridization

The embryo tissues for in situ hybridization were fixed freshly prepared 4% paraformaldehyde in DEPC-treated PBS, dehydrated, processed through a standard paraffin embedding protocol under RNase-free condition. Digoxigenin labeled non-radioactive in situ hybridization was performed on 8 um paraffin sections ofE13.5d embryo as described previously (Kishimoto J, Cox H, Deverne EB, and Emson PC "Cellular Localization of Putative Odorant Receptor Messenger RNAs in Olfactory and Chemosensory Neurons - A Non Radioactive Insitu Hybridization Study" *Molecular Brain Research* 23:33-39 (1994)). CDNA for lacZ and mouse versican antisense probe were prepared by PCR and sub-cloned into pBluescriptII(stratagene). Digoxigenin labeled RNA probes were prepared using a RNA labeling kit (Boehringer) according to the manufacturers' instruction. Paraffin sections on slides were quickly dewaxed in xylene, dehydrated through 100%, 90%, 70% ethanol, washed with O.1M PBS (pH7.5) and then treated with 0.2N hydrochloric acid for 10 minutes. Sections were treated with 0.02% pepsin (in 0.2N HCl) for 30 minutes at 37°C, following which the enzyme was deactivated by 4% paraformaldehyde. After rinsing in 0.2% glycine solution (in PBS) three times, slides were acetylated in 0.25% acetic anhydride in 0.1 M triethanolamine / 0.9% NaCl for 10 minutes at room temperature and partially dehydrated through 70, 80, 90% ethanol and briefly dried. Approximately 0.1 µg/ml digoxigenin labeled RNA probes (denatured by boiling prior to use) were added in hybridization buffer (50% deionized formamide, 4XSSC, 10% dextran sulphate, 1X Denhardt's solution) and slides were incubated at 55°C under coverslips for 16-18 hours. The coverslips were carefully removed and the sections were washed in 2X SSC/0.1% SDS for 30 minutes at room temperature, sequentially washed twice in O.1X SSC / 0.1% SDS at 60°C for 30 minutes. Immunohistochemical procedures to visualize the hybridized probes used alkaline phosphatase conjugated anti-manufactures' instructions. The colorimetric reaction was started by adding nitroblue tetrazolium (Boehringer) and 5-bromo-4-chloro-3-indolyl-phosphate (Boehringer) and incubated for 6-24 hours at room temperature. Slides were mounted with glycerin jelly and were kept at 4°C.

Radioactive in situ hybridization with 3' end labeled oligo nucleotide probes was carried out as described previously (Kishimoto J. Keverne EB, Hardwick J. Emson PC, "Localization of nitric oxide synthase in the mouse olfactory and vomeronasal system: a histochemical, immunological and in situ hybridization study" *European J. of Neuroscience* 5:1684-1694 (1993)). Sequences of mouse versican antisense oligonucleotide probes were 5'-CCGTTCTGGGCCACCAAGACAGTCGTCTCC-3', 5'-TGACCGCCCCGATATCCAAACAAGCCTGTT-3' (SEQ ID NO: _), 5'-TCCGACAGCCAGCCGTAATCGCATTGGTCA-3' (SEQ ID NO: _). Some sections were hybridized with labeled oligonucleotides in the presence of excess unlabelled same oligonucleotides to check the specificity of the *in situ* signal.

### Cell culture

For primary fibroblast culture from transgenic mice skin, newborn transgenic mice skin were pealed off and incubated in 0.25% trypsin 18 hrs. Discarded epidermal sheet and dermis were further digested by collagenase for 1 hr. Cells were spread out to the MEDM medium (GIBCO)+ 10% FCS. cells were passaged in every 3 days after trypsin treatment. For b-galactosidase histochemistry cultured fibroblast cells were fixed with 0;.2% glutaraldehyde in PBS for 2 min., followed by detergent wash solution for 5 min. and staining reaction was performed for 18 hrs. at 30°C. After reaction was stopped cells were proceeded for counter-nuclear staining with 5µg/ml of 7-aminoactinomycin D (SIGMA, USA) solution for 20 min. at R.T. then, washed PBS, mount with fluoromount-G (Southern Biotechnology, AL).

### Generation of hairless versican-lacZ transgenic mice

To eliminate hair follicle derived lacZ expression in order to examine age-related change of versican promoter activity, hairless genetic background of versican-lacz transgenic mice were obtained by breeding with SKH-1 hairless mice resultant founder with SKH-1 (Charles River Laboratories, Boston). Thus, male hemizygous transgenic mice (v/-HH) were mated with female hairless mice(-/-hh), and selected transgene positive pups (v/-Hh) by genomic PCR were then mated back to a hairless again to obtain some F2 generation of versican-lacZ hairless mice(v/-hh) which can be easily selected by combination of PCR detection and hairless appearance.

### Generation of versican-lacZ transgenic mice

Of a total of 27 founder mice six positive lines were detected by both PCR and southern blotting analysis for the transgene insertion. The copy number of transgene was 1 to more than 20 copies per cell. These were further screened for lacZ staining by using X-gal histochemical reaction on tail skin. The intensity of lacZ staining in tail did not correlated with the copy number of transgene as both two lines. A4681(1-2 copies) and A4688(over 20 copies) exhibited similar strong lacZ staining in tail hairs with A4679 which had also over 20 copies, showed only light staining in tail. The transgenic line A4681 was chosen for further study as the staining of tail skin was most intense and had a good breeding behavior. All further analysis was performed on F1-F3 offspring derived by mating with DBA/2 strain.

### Distribution of lacZ expression in embryonic tissue

Intense lacZ staining was observed in developing fore and hind limb (E13.5) within an area of mesenchymal condensation and also in ectoderm. This ectodermal expression was restricted in the tip of limb. In situ hybridization with radio-labeled antisense oligoes showed similar endogenous mouse versican mRNA expression in condenses mesenchyme in limb bud.

In E15.5 embryo strong lacZ staining was also observed in kidney glomeruli mesenchyme in olfactory epithelium, mesenchyme and muscles in tongue, and submandibular gland. Perichondrocyte surrounding cartilage, blood vessel, developing edge of hind and fore brain, smooth muscle were also positive.

The expected expression pattern other than limb was confirmed by digoxigenin non-radioactive in situ hybridization with lacZ and endogenous mouse versican probes on transgenic embryonic section (E13.5) as well as control sense probes. Both mRNA expression was observed in, kidney, olfactory epithelium, humerus cartilage, and in vertebrae cartilage, confirming reasonable expression pattern and timing of expression of this transgenic mice.

### Human versican activity in embryonic skin

LacZ expression was examined by X-gal histochemistry on developing skin from E13.5, E14.5, E15.5 and E17.5 embryo. At E13.5d basically no staining was observed in whole body of ectoderm except hind and fore limb and with a few occasion trace of lacZ staining was found in single mesenchymal cell which seems to be earliest stage of condensation. Hair germ of whisker has already exhibited lacZ staining at this stage. At E14.5 condensed mesenchymal cells attached beneath ectodermal placode (hair plug) were clearly lacZ positive and this was highly contrasted against complete negative surrounding scattered mesenchymal cells. Phase contrast photo showed individual b-gal positive condensed mesenchymal cell. The number of these positive site were 4 -5 per sagittal - mediaeval embryonic section of whole embryo tissue. At E15.5 these positive condenses mesenchyme under hair plug were getting more intense and increased in volume (i.e. the number of cell). At E 17.5 the number and intensity of lacZ positive dermal papilla are dramatically increased, yet still highly restricted within the future dermal papilla. A few epidermal placode (i.e. epithelial keratinocytes) itself exhibited lacZ staining although the majority were still restricted in mesenchymal cells.

### Hair cycle dependent human versican activity in transgenic mice skin

LacZ staining was examined on the skin section of transgenic mice from newborn, 7 days, 14 days, 28 days, 40 days, to 4 month old. Strong lacZ staining in hair dermal papilla cells in late embryonic skin were proceed into new born skin and continue during first anagen hair cycle. Strong staining was confined within the dermal papilla cells. Interestingly at age 7, mid to late anagen stage relatively strong staining was also observed in inner root sheath. However, in late anagen stage lacZ staining was again restricted only in dermal papilla cells. Also later in telogen hair no lacZ staining observes in club hair. In second hair cycle growing dermal papilla (second anagen hair cycle) exhibited again intense lacZ staining and after second anagen hair cycle lacZ activity in hair dermal papilla was decreased almost completely through long telogen hair cycle.

### Human versican activity in cultures fibroblast

Primary dermal fibroblast culture derived from dermal fibroblast cells of new born transgenic line exhibited lacZ staining in only certain population of cells and center of hair clamp, obviously dermal papilla. Since all strongly stained cells were round shape these should be derived hair dermal papilla. After first passage, however, shape of positive cells were diversed and not confined round shape, including typical fibroblast-like cells with dendritic process in passage 2. These population of positive cells maintained after first passage to at least until six passages we cultured while certain number of cells were completely unstained through passages determined with counter-nuclear staining which clearly indicated total number of cells. Approximate number of positive cells were not exceed over 50%. Intensity of staining was gradually faded with passages.

### Age-related change of human versican activity in transgenic mouse skin

Increasing reaction time for x-gal staining to 18 hrs revealed more staining in upper dermis other than hair dermal papilla in young to adult skin of the transgenic mouse. In newborn mouse skin under this long incubation condition, most of dermis including hair follicle (hairless mouse have normal hair only in first cycle) showed fairly detectable staining. Young mouse skin showed only isolated fibroblast derived diffused staining preferentially in upper dermis, and older mouse only exhibited faint staining, indicating age-related decrease of human versican promoter activity in transgenic mouse skin.

### Example 8: Evaluation of Reporter gene expression in a live animal. Method:

### Confocal microscopy

To examine whole intact skin on living transgenic mouse with confocal microscope, hairless VEGF-GFP transgenic mice were anaesthetized with avertin by intraperitoneal injection. They were placed directly on the petri dish with dorsal position. TPA or acetone treatment was performed on the back skin same as described above marking treated area with an ink to ease the orientation of the skin. Sections or intact skin of the mice were analyzed using Leica DM IRBE inverted microscope and Leica TCS NT4D confocal microscope system with band pass filter 530/30 nm, detecting emission at wavelengths between 515-545 nm.

### Direct detection of GFP in the epidermis of intact skin of living hairless transgenic mice by confocal microscopy

To test whether the induction of GFP is directly detected in the skin of a living VEGF-GFP transgenic mouse, acetone or TPA applied skin of the transgenic *hairless* mouse (same individual) was directly examined by confocal imaging under anaesthetized condition. Horizontal optical sections through the normal living epidermis revealed detectable fluorescence and the skin treated with TPA after 12hrs showed the increase of fluorescence positive area. Non-transgenic wild type litter mate showed no detectable specific fluorescence. Higher magnification of the TPA treated skin revealed that GFP was localized in the extracellular space around the keratinocytes. X-Z confocal imaging confirmed that this signal was within the basal layer of the epidermis.

Laser confocal microscopic evaluation of an intact skin of living transgenic mouse for detection of the GFP signal indicates that GFP is readily detected within the epidermis. These results suggest the potential use of GFP transgenic mice for non-invasive monitoring of long-term gene expression *in vivo.* When the transgenic GFP reporter gene is combined with the technology for microscopic evaluation such as confocal microscope, the system has a great advantage over conventional transgenic reporter gene animals methods because it reduces the number of animals used, utilizes a simple monitoring system, and allows long term monitoring of changes in gene expression on the same individual.

Successful *in vivo* expression of GFP in transgenic mouse skin provides for the understanding of the regulation of VEGF gene expression, such as in inflammatory and neoplastic skin diseases, by providing a useful monitoring and screening tool. It is particularly attractive when bred to a mouse with a phenotype which involves VEGF expression. This novel experimentation model will allow in vivo studies on the regulation of VEGF gene. This GFP reporter system on transgenic animal would also be advantageous in research focused on skin homeostasis during the aging process.

### Example 9: human MMP9 transgenic mice

MMP9 (gelatinase B, 92-kDa type IV collagenase) is one of the member of matrix metalloproteinases (MMPs) capable to degrade extracellular matrix (ECM) components. This enzyme (MMP9) is known to degrade type IV and V collagens, gelatin and elastin. It is also shown to be induced by UVB which causes photoaging. (Fisher, G.J., *Nature* **379**, 335, 1996). 5'flanking region of this gene from -670 sequence position to +1 transcription start site is sufficient to drive expression of a reporter gene (CAT) in HT1080 cells. (Sato, H., *Oncogene* **8**, 395, 1993).

### Construct of the Transgene

To construct the transgene, an 733 bp fragment of the human MMP9 promoter (nucleotides -714 to +19; the transcription start site being +1) was obtained from human genomic DNA using art known PCR-based techniques. Two different reporter genes, beta-galactosidase reporter gene (lacZ) and green fluorescent protein (GFP) gene, were employed for construction.

For the construction with beta-galactosidase reporter gene (lacZ), the MMP9 promoter fragment was inserted into an modified pNASSbeta vector (Clontech), which is inserted neutrophil elastase promoter and has Bgl II site, linearized by restriction with Bgl II-Xho I. To obtain transgene DNA fragment for pronuclear injection, the vector was cleaved with Bgl II and Hind III. The length of the linearized transgene DNA was 4630bp.

For the construction with green fluorescent protein (GFP) gene, the same MMP9 promoter fragment was inserted into the pEGFP-1-SV, which was modified vector to have splice donor/acceptor based on pEGFP-1 vector (Clontech), linearized by restriction with Hind III-Kpn I. To obtain transgene DNA fragment for pronuclear injection, the vector was cleaved with Hind III and Afl II. The length of the linearized transgene DNA was 1928bp.

### Generation of transgenic mice

The lenearized transgene DNA fragment was injected into fertilized oocytes of hairless mice (SKH1; Charles River, Boston). Those eggs were implanted into pseudo pregnant foster mothers. The offsprings (F0 were tested for chromosome integration of lacZ or GFP fragment by southern blotting. For the detection of lacZ transgene, the genomic DNA extracted from the tail was digested with Nci I and separated on a gel, then transferred on a nylon membrane. The Nci I digested transgene DNA fragment was used for the probe. Two independent lines showed a transgene insertion.
For the detection of GFP transgene, the tail genomic DNA was digested with Hinc II Two independent lines showed a transgene insertion.

### beta-galactosidase histochemistry

F1 mice obtained from founders (FO) were used for beta-galactosidase histochemistry. Bones of 2-wk-old mice hindlimb were fixed with 0.5% glutaraldehyde in PBS for 30 minutes. After three times washing with PBS and once with detergent wash, the tissue was incubated with reaction buffer containing 1 mg/ml X-gal (Sigma), 10 mM ferrocyanide and 10 mM ferricyanide at 37°C. After 2 hours incubation, blue staining was observed in a F1 mouse which has transgene, while no staining was observed in the sibling without transgene. These are consistent with a report in which MMP9 was shown to express at bone of 2-wk-old mouse limb by in situ hybridization (Reponen, P., *Journal of Cell Biology* **124**, 1091, 1994).

### Example 10: human Neutrophil Elastase transgenic mice

Neutrophil elastase is one of the powerful serine proteinase capable attacking a broad range of proteins. Hairless mice deficient in this enzyme do not suffer from elastosis, which is one of signs of photoaging caused by UVB irradiation. (Starcher, B., *Connective Tissue Research,* **31**, 133, 1995). Therefore, neutrophil elastase is believed to play an important roll in photoaging.

### Construction of the Transgene

To construct the transgene, an 1299 bp fragment of the human neutrophil elastase promoter (nucleotides -1280 to +19; the transcription start site being +1) was obtained from human genomic DNA using art known PCR-based techniques. This neutrophil elastase promoter fragment was inserted into the pNASSbeta vector (Clontech) linearized by restriction with Eco RI - Xho I. To obtain transgene-DNA fragment for pronuclear injection, the vector was cleaved with EcoR I and Xba I. The length of the linearized transgene DNA was 5171bp.

## Claims

1. A non-human transgenic animal having a reporter gene coupled to a versican promoter.

2. The non-human transgenic animal of claim 1, wherein the versican promoter is a human versican promoter.

3. The transgenic animal of claim 1, wherein the reporter gene is selected from the group consisting of the genes which encode beta-galactosidase, alkaline phosphatase, horseradish peroxidase, and chloramphenicol-acetyltransferase.

4. The non-human transgenic animal of claim 1, wherein the reporter gene produces a gene product which is fluorescent, or which catalyzes a reaction which can be determined by formation of a colored product.

5. The transgenic animal of claim 4, wherein the reporter gene produces a gene product which is fluorescent.

6. The transgenic animal of claim 4, wherein the reporter gene is green fluorescent protein.

7. The transgenic animal of claim 1, wherein the transgenic animal is a mouse.

8. The transgenic mouse of claim 7, wherein the mouse is a hairless mouse.

9. The transgenic mouse of claim 7, wherein the mouse is a senescence accelerated mouse.

10. The transgenic animal of claim 1, wherein the transgenic animal further includes a second reporter gene coupled to a skin metabolism-related promoter different from the versican promoter.

11. A method of evaluating a treatment for its effect on skin, comprising:
a. providing tissues taken from the transgenic animal of claim 1 or 10;
b. administering the treatment to the tissues; and
c. evaluating expression of the reporter gene; thereby evaluating the treatment for its effect on skin.

12. A method of evaluating a treatment for its effect on skin, comprising:
a. providing the transgenic animal of claim 1 or 10 or tissues thereof;
b. administering the treatment to the transgenic animal or the tissues; and
c. evaluating expression of the reporter gene; thereby evaluating the treatment for its effect on skin;
wherein the transgenic animal is a mouse, and the treatment is administered to the animal; or wherein the transgenic animal is a rat; or wherein the transgenic animal is a guinea pig.

13. The method of claim 1 or 12 wherein the treatment is applied to the skin of the transgenic animal.

14. The method of claim 11 or 12 wherein the versican promoter is a human versican promoter.

15. The method of claim 11 or 12 wherein the reporter gene is selected from the group consisting of the genes which encode beta-galactosidase, alkaline phosphatase, horseradish peroxidase, and chloramphenicol-acetyltransferase.

16. The method of claim 11 or 12 wherein the reporter gene produces a gene product which is fluorescent, or which catalyzes a reaction which can be determined by formation of a colored product.

17. The method of claim 16, wherein the reporter gene produces a gene product which is fluorescent.

18. The method of claim 16, wherein the reporter gene is green fluorescent protein.

19. The method of claim 12, wherein the mouse is a hairless mouse.

20. The method of claim 12, wherein the mouse is a senescence accelerated mouse.

21. The method of claim 11 or 12 wherein the transgenic animal is a rat.

22. The method of claim 11 or 12, wherein the transgenic animal is a guinea pig.

23. The method of claim 11 or 12, wherein the transgenic animal further includes a second reporter gene coupled to a skin metabolism-related promoter different from the versican promoter.

24. The method of claim 12, wherein the treatment is administered to the animal, and further comprising a subsequent administration of the treatment.

25. The method of claim 11 or 12, further comprising comparing the expression of the reporter gene to a control value.

26. The method of claim 12, wherein the treatment is administered to the animal, and the evaluation of the expression of the reporter gene step is repeated at least once during the life of the animal.

27. The method of claim 26, wherein the first and a subsequent repetition of the step can be separated by as much as 10 days.

28. The method of claim 26, wherein the first and a subsequent repetition are performed on a live animal.

29. The method of claim 28, wherein the evaluation of the expression of the reporter gene in the transgenic animal comprises evaluating expression of the reporter gene in the skin of the transgenic animal without removing a sample of skin from the transgenic animal.

30. The method of claim 12, wherein the evaluation of the expression of the reporter gene comprises removing a sample of skin from the transgenic animal.

31. The method of claim 30, wherein the sample of skin is alive during the evaluation of the expression of the reporter gene.

## Patentansprüche

1. Ein nicht menschliches transgenes Tier mit einem an einen Versican-Promotor gekoppelten Reportergen.

2. Das nicht menschliche transgene Tier nach Anspruch 1, wobei der Versican-Promotor ein menschlicher Versican-Promoter ist.

3. Das transgene Tier nach Anspruch 1, wobei das Reportergen aus der Gruppe ausgewählt wird, die aus den Genen besteht, die Beta-Galactosidase, Alkalinphosphatase, Meerrettich-Peroxidase und Chloramphenicol-Acetyltransferase kodieren.

4. Das nicht menschliche transgene Tier nach Anspruch 1, wobei das Reportergen ein Genprodukt herstellt, das fluoreszierend ist, oder das eine Reaktion katalysiert, die durch die Bildung eines farbigen Produkts bestimmt wird.

5. Das transgene Tier nach Anspruch 4, wobei das Reportergen ein Genprodukt herstellt, das fluoreszierend ist.

6. Das transgene Tier nach Anspruch 4, wobei das Reportergen ein grün fluoreszierendes Protein ist.

7. Das transgene Tier nach Anspruch 1, wobei das transgene Tier eine Maus ist.

8. Das transgene Tier nach Anspruch 7, wobei die Maus eine haarlose Maus ist.

9. Das transgene Tier nach Anspruch 7, wobei die Maus eine Maus ist, bei der die Vergreisung beschleunigt ist.

10. Das transgene Tier nach Anspruch 1, wobei das transgene Tier außerdem ein zweites Reportergen umfasst, das an einen auf den Haut-Metabolismus bezogenen Promoter gekoppelt ist, der sich vom Versican-Promoter unterscheidet.

11. Ein Verfahren zur Auswertung der Wirkung einer Behandlung auf die Haut, umfassend:
a. Bereitstellung von aus dem transgenen Tier nach dem Patentanspruch 1 oder 10 entnommenen Geweben;
b. Verabreichung der Behandlung an die Gewebe; und
c. Auswertung der Expression des Reportergens; wobei die Behandlung auf ihre Wirkung auf die Haut untersucht wird.

12. Ein Verfahren zur Auswertung der Wirkung einer Behandlung auf die Haut, umfassend:
a. Bereitstellung des transgenen Tiers nach dem Patentanspruch 1 oder 10, oder von Geweben davon;
b. Verabreichung der Behandlung an das transgene Tier oder Gewebe davon; und
c. Auswertung der Expression des Reportergens; wobei die Behandlung auf ihre Wirkung auf die Haut untersucht wird;
wobei das transgene Tier eine Maus ist, und die Behandlung dem Tier verabreicht wird; oder wobei das transgene Tier eine Ratte ist; oder wobei das transgene Tier ein Meerschweinchen ist.

13. Das Verfahren nach Anspruch 11 oder 12, wobei die Behandlung auf die Haut des transgenen Tiers angewandt wird.

14. Das Verfahren nach Anspruch 11 oder 12, wobei der Versican-Promotor ein menschlicher Versican-Promoter ist.

15. Das Verfahren nach Anspruch 11 oder 12, wobei das Reportergen aus der Gruppe ausgewählt wird, die aus den Genen besteht, die Beta-Galactosidase, Alkalinphosphatase, Meerrettich-Peroxidase und Chloramphenicol-Acetyltransferase kodieren.

16. Das Verfahren nach Anspruch 11 oder 12, wobei das Reportergen ein Genprodukt herstellt, das fluoreszierend ist, oder das eine Reaktion katalysiert, die durch die Bildung eines farbigen Produkts bestimmt wird.

17. Das Verfahren nach Anspruch 11 oder 12, wobei das Reportergen ein Genprodukt herstellt, das fluoreszierend ist.

18. Das Verfahren nach Anspruch 16, wobei das Reportergen ein grün fluoreszierendes Protein ist.

19. Das Verfahren nach Anspruch 12, wobei die Maus eine haarlose Maus ist.

20. Das Verfahren nach Anspruch 12, wobei die Maus eine Maus ist, bei der die Vergreisung beschleunigt ist.

21. Das Verfahren nach Anspruch 11 oder 12, wobei das transgene Tier eine Ratte ist.

22. Das Verfahren nach Anspruch 11 oder 12, wobei das transgene Tier ein Meerschweinchen ist.

23. Das Verfahren nach Anspruch 11 oder 12, wobei das transgene Tier außerdem ein zweites Reportergen umfasst, das an einen Haut-Metabolismus bezogenen Promoter gekoppelt ist, der sich vom Versican-Promoter unterscheidet.

24. Das Verfahren nach Anspruch 12, wobei die Behandlung dem Tier verabreicht wird, und außerdem eine nachfolgende Verabreichung der Behandlung umfasst.

25. Das Verfahren nach Anspruch 11 oder 12, umfassend außerdem den Vergleich der Expression des Reportergens mit einem Kontrollwert.

26. Das Verfahren nach Anspruch 12, wobei die Behandlung dem Tier verabreicht wird, und die Auswertung der Expression des Reportergenschritts mindestens einmal im Leben des Tiers wiederholt wird.

27. Das Verfahren nach Anspruch 26, wobei der erste Schritt und eine folgende Wiederholung des Schritts durch 10 Tage getrennt sein können.

28. Das Verfahren nach Anspruch 26, wobei der erste Schritt und eine folgende Wiederholung des Schritts an einem lebenden Tier ausgeführt werden.

29. Das Verfahren nach Anspruch 28, wobei die Auswertung der Expression des Reportergens im transgenen Tier die Auswertung der Expression des Reportergens in der Haut des transgenen Tiers ohne Entnahme einer Hautprobe vom transgenen Tier umfasst.

30. Das Verfahren nach Anspruch 12, wobei die Auswertung der Expression des Reportergens die Entnahme einer Hautprobe vom transgenen Tier umfasst.

31. Das Verfahren nach Anspruch 30, wobei die Hautprobe während der Auswertung der Expression des Reportergens am Leben bleibt.

## Revendications

1. Animal transgénique non-humain ayant un gène rapporteur couplé à un promoteur du versicane.

2. Animal transgénique non-humain selon la revendication 1, dans lequel le promoteur du versicane est un promoteur du versicane humain.

3. Animal transgénique selon la revendication 1, dans lequel le gène rapporteur est sélectionné dans le groupe constitué des gènes qui codent pour une bêta-galactosidase, une phosphatase alcaline, une péroxydase de raifort, et une chloramphénicol-acétyltransférase.

4. Animal transgénique non-humain selon la revendication 1, dans lequel le gène rapporteur produit un produit de gène qui est fluorescent, ou qui catalyse une réaction qui peut être déterminée par formation d'un produit coloré.

5. Animal transgénique selon la revendication 4, dans lequel le gène rapporteur produit un produit de gène qui est fluorescent.

6. Animal transgénique selon la revendication 4, dans lequel le gène rapporteur est une protéine fluorescente verte.

7. Animal transgénique selon la revendication 1, dans lequel l'animal transgénique est une souris.

8. Souris transgénique selon la revendication 7, dans laquelle la souris est une souris hairless.

9. Souris transgénique selon la revendication 7, dans laquelle la souris est une souris à sénescence accélérée.

10. Animal transgénique selon la revendication 1, dans lequel l'animal transgénique inclut en outre un second gène rapporteur couplé à un promoteur associé au métabolisme de la peau différent du promoteur du versicane.

11. Procédé d'évaluation de l'effet d'un traitement sur la peau, comprenant:
a. le fait de fournir des tissus prélevés sur l'animal transgénique selon la revendication 1 ou 10;
b. l'administration du traitement aux tissus; et
c. l'évaluation de l'expression du gène rapporteur, évaluant ainsi l'effet du traitement sur la peau.

12. Procédé d'évaluation de l'effet d'un traitement sur la peau, comprenant:
a. le fait de fournir l'animal transgénique selon la revendication 1 ou 10 ou des tissus de celui-ci;
b. l'administration du traitement à l'animal transgénique ou aux tissus; et
c. l'évaluation de l'expression du gène rapporteur, évaluant ainsi l'effet du traitement sur la peau;
dans lequel animal transgénique est une souris, et le traitement est administré à l'animal; ou dans lequel animal transgénique est un rat; ou dans lequel l'animal transgénique est un cobaye.

13. Procédé selon la revendication 11 ou 12, dans lequel le traitement est appliqué à la peau de l'animal transgénique.

14. Procédé selon la revendication 11 ou 12, dans lequel le promoteur du versicane est un promoteur du versicane humain.

15. Procédé selon la revendication 11 ou 12, dans lequel le gène rapporteur est sélectionné dans le groupe constitué des gènes qui codent pour une bêta-galactosidase, une phosphatase alcaline, une péroxydase de raifort, et une chloramphénicol-acétyltransférase.

16. Procédé selon la revendication 11 ou 12, dans lequel le gène rapporteur produit un produit de gène qui est fluorescent, ou qui catalyse une réaction qui peut être déterminée par formation d'un produit coloré.

17. Procédé selon la revendication 16, dans lequel le gène rapporteur produit un produit de gène qui est fluorescent.

18. Procédé selon la revendication 16, dans lequel le gène rapporteur est une protéine fluorescente verte.

19. Procédé selon la revendication 12, dans laquelle la souris est une souris hairless.

20. Procédé selon la revendication 12, dans laquelle la souris est une souris à sénescence accélérée.

21. Procédé selon la revendication 11 ou 12, dans lequel l'animal transgénique est un rat.

22. Procédé selon la revendication 11 ou 12, dans lequel animal transgénique est un cobaye.

23. Procédé selon la revendication 11 ou 12, dans lequel l'animal transgénique inclut en outre un second gène rapporteur couplé à un promoteur associé au métabolisme de la peau différent du promoteur du versicane.

24. Procédé selon la revendication 12, dans lequel le traitement est administré à l'animal, et qui comprend en outre une administration ultérieure du traitement.

25. Procédé selon la revendication 11 ou 12, qui comprend en outre la comparaison de l'expression du gène rapporteur à une valeur témoin.

26. Procédé selon la revendication 12, dans lequel le traitement est administré à l'animal, et l'étape d'évaluation de l'expression du gène rapporteur est répétée au moins une fois pendant la vie de l'animal.

27. Procédé selon la revendication 26, dans lequel la première étape et une répétition ultérieure de l'étape peuvent être séparées par jusqu'à 10 jours.

28. Procédé selon la revendication 26, dans lequel la première étape et une répétition ultérieure de l'étape sont réalisées sur un animal vivant.

29. Procédé selon la revendication 28, dans lequel l'évaluation de l'expression du gène rapporteur chez l'animal transgénique comprend l'évaluation de l'expression du gène rapporteur au niveau de la peau de l'animal transgénique sans prélever un échantillon de peau de l'animal transgénique.

30. Procédé selon la revendication 12, dans lequel l'évaluation de l'expression du gène rapporteur comprend le prélèvement d'un échantillon de peau de l'animal transgénique.

31. Procédé selon la revendication 30, dans lequel échantillon de peau est vivant pendant l'évaluation de l'expression du gène rapporteur.
